(19) Europäisches Patentamt
European Patent Office
Office européen des brevets

(11) Publication number: **0 333 356 A2**

(12) # EUROPEAN PATENT APPLICATION

(21) Application number: 89302160.0

(22) Date of filing: 03.03.89

(51) Int. Cl.4: C07K 7/00 , A61K 37/00

(30) Priority: 04.03.88 US 164178
29.09.88 US 251150

(43) Date of publication of application:
20.09.89 Bulletin 89/38

(84) Designated Contracting States:
AT BE CH DE ES FR GB GR IT LI LU NL SE

(71) Applicant: BIOGEN, INC.
14 Cambridge Center
Cambridge Massachusetts 02142(US)

(72) Inventor: Maraganore, John M.
84 Patrick Road
Tewksbury Massachusetts 01876(US)

(74) Representative: Bannerman, David Gardner et al
Withers & Rogers 4 Dyer's Buildings Holborn
London, EC1N 2JT(GB)

(54) **Hirudin peptides.**

(57) This invention relates to novel, biologically active peptides which display the anticoagulant activity of hirudin. More specifically, it relates to peptides which are homologous to at least a portion of the carboxy terminal 26 amino acids of hirudin. Such peptides may also be characterized by a modified tyrosine residue. This invention further relates to covalent and peptidomimetic analogs of these peptides which display anticoagulant activity. This invention also relates to compositions, combinations and methods using such peptides or analogs for therapeutic, prophylactic or diagnostic purposes. And this invention relates to novel methods for sulfating tyrosine residues contained within a peptide or polypeptide.

EP 0 333 356 A2

Xerox Copy Centre

# HIRUDIN PEPTIDES

## TECHNICAL FIELD OF INVENTION

This invention relates to novel, biologically active peptides which display the anticoagulant activity of hirudin. More specifically, it relates to peptides which are homologous to at least a portion of the carboxy terminal 26 amino acids of hirudin. Such peptides may also be characterized by a modified tyrosine residue. This invention further relates to peptidomimetic and covalent analogs of these peptides which display anticoagulant activity. This invention also relates to compositions, combinations and methods using such peptides or analogs for therapeutic, prophylactic or diagnostic purposes. And this invention relates to novel methods for sulfating tyrosine residues contained within a peptide or polypeptide.

## BACKGROUND ART

Acute vascular diseases, such as myocardial infarction, stroke, pulmonary embolism, deep vein thrombosis, peripheral arterial occlusion, and other blood system thromboses constitute major health risks. Such diseases are caused by either partial or total occlusion of a blood vessel by a blood clot, which consists of fibrin.

Current methods for treatment and prophylaxis of thrombotic diseases involve therapeutics which act in one of two different ways. The first type of therapeutic inhibits thrombin activity or thrombin formation, thus preventing clot formation. These drugs also inhibit platelet activation and aggregation. The second category of therapeutic accelerates thrombolysis and dissolves the blood clot, thereby removing it from the blood vessel and unblocking the flow of blood [J. P. Cazenave et al., Agents Action, 15, Suppl., pp. 24-49 (1984)].

Heparin, a compound of the former class, has been widely used to treat conditions, such as venous thromboembolism, in which thrombin activity is responsible for the development or expansion of a thrombus. Although effective, heparin produces many undesirable side effects, including hemorrhaging and thrombocytopenia. This has led to a search for a more specific and less toxic anticoagulant.

Hirudin is a naturally occurring polypeptide which is produced by the blood sucking leech Hirudo medicinalis. This compound, which is produced in the salivary gland of the leech, is the most potent natural inhibitor of coagulation known. Hirudin prevents blood from coagulating by binding tightly to thrombin ($K_d$ ~ 2 x $10^{-11}$M) in a 1:1 stoichiometric complex [S. R. Stone and J. Hofsteenge, "Kinetics of the Inhibition of Thrombin by Hirudin", Biochemistry, 25, pp. 4622-28 (1986)]. This, in turn, inhibits thrombin from catalyzing the conversion of fibrinogen to fibrin (clot).

Hirudin is believed to be a selective and specific inhibitor of thrombin which exhibits no inhibitory activities toward other coagulation factors. It has been suggested, however, that hirudin inhibits both Factor IXa activation of Factor X and Factor Xa activation of prothrombin [E. W. Davie et al., "Blood Coagulation", Adv. Enzymol., 48, pp. 277-318 (1979); J. S. Rosenberg et al., "Activation of Human Prothrombin by Highly Purified Human Factors V and Xa in the Presence of Human Antithrombin", J. Biol. Chem., 250, pp. 1607-1617 (1975)]. Recently, it has been demonstrated that purified hirudin accelerates the displacement of $^{125}$I-labeled Factor Xa from cultured endothelial cells; that $^{125}$I-labeled hirudin binds to Factor Xa; and that purified hirudin does not inhibit Factor Xa cleavage of the small chromogenic substrate, S-2222 [R. C. Friedberg et al., "The Role of Endothelium in Factor Xa Regulation: The Effect of Plasma Proteinase Inhibitors and Hirudin", Blood, 71, pp. 1321-28 (1988)].

None of these studies of hirudin, however, rules out the possibility that a protein contaminant with Factor Xa inhibitory activity may have been present in the preparations used. Accordingly, to date, there is no conclusive evidence that hirudin or a hirudin-related structure inhibits Factors IXa and Xa. One possible contaminant in previously studied hirudin preparations may be the equivalent of antistasin, a protein which has been purified from the Mexican leech, Haementeria officinalis, and which selectively inhibits Factor Xa [G. P. Tuszynski et al., "Isolation and Characteristics of Antistasin", J. Biol. Chem., 262, p. 9718 (1987)]. However, antistasin, unlike hirudin, inhibits the cleavage of S-2222 by Factor Xa. This implies that the inhibition of Factor Xa by hirudin occurs via a different mechanism than the Factor Xa inhibition by antistasin.

The actual binding between hirudin and thrombin is a two-step process. Initially, hirudin binds to a "low" affinity site on the thrombin molecule ($K_d$ ~ 1 x 10 $^{-8}$M) which is separate from the catalytic site.

Following the low affinity binding, hirudin undergoes a conformational change and then binds to the "high" affinity site on thrombin. This latter site corresponds to the active site of thrombin.

The isolation, purification and chemical composition of hirudin are known in the art [P. Walsmann and F. Markwardt, "Biochemical and Pharmacological Aspects of the Thrombin Inhibitor Hirudin", Pharmazie, 36, pp. 653-60 (1981)]. More recently, the complete amino acid sequence of the polypeptide has been elucidated [J. Dodt et al. "The Complete Covalent Structure of Hirudin: Localization of the Disulfide Bonds", Biol. Chem. Hoppe-Seyler, 366, pp. 379-85 (1985); S. J. T. Mao et al., "Rapid Purification and Revised Amino Terminal Sequence of Hirudin: A Specific Thrombin Inhibitor of the Blood-Sucking Leech", Anal. Biochem, 161, pp. 514-18 (1987); and R. P. Harvey et al., "Cloning and Expression of a cDNA Coding for the Anti-Coagulant Hirudin from the Bloodsucking Leech, Hirudo medicinalis", Proc. Natl. Acad. Sci. USA, 83, pp. 1084-88 (1986)].

At least two different isospecific forms of hirudin, HV-1 and HV-2, have been sequenced and have been shown to differ slightly in amino acid sequence [R. P. Harvey et al., supra]. Both forms of hirudin comprise a single polypeptide chain protein containing 65 amino acids in which the amino terminus primarily comprises hydrophobic amino acids and the carboxy terminus typically comprises polar amino acids. More specifically, all forms of hirudin are characterized by an N-terminal domain (residues 1-39) stabilized by three disulfide bridges in a 1-2, 3-5, and 4-6 half-cysteinyl pattern and a highly acidic C-terminal segment (residues 40-65). In addition, the C-terminal segment of hirudin is characterized by the presence of a tyrosine residue at amino acid position 63 which is sulfated.

In animal studies, hirudin, purified from leeches, has demonstrated efficacy in preventing venous thrombosis, vascular shunt occlusion and thrombin-induced disseminated intravascular coagulation. In addition, hirudin exhibits low toxicity, little or no antigenicity and a very short clearance time from circulation [F. Markwardt et al., "Pharmacological Studies on the Antithrombotic Action of Hirudin in Experimental Animals", Thromb. Haemostasis, 47, pp. 226-29 (1982)].

Despite hirudin's effectiveness, however, studies have shown that hirudin prolongs bleeding time in a dose-dependent manner, thus making the determination and administration of proper dosages critically important. Furthermore, the high cost and low supply of the naturally occurring product has prevented its widespread use.

In an effort to create a greater supply of hirudin, attempts have been made to produce the polypeptide through recombinant DNA techniques. The presence of an O-sulfated tyrosine residue on native hirudin and the inability of microorganisms to perform a similar protein modification made the prospect of recombinant production of biologically active hirudin highly speculative. The observation that desulfato-hirudins were almost as active as their sulfated counterparts [United States patent 4,654,302], however, led the way to the cloning and expression of hirudin in E.Coli [European patent applications 158,564, 168,342 and 171,024] and yeast [European patent application 200,655]. Despite these advances, hirudin is still somewhat expensive to produce and it is not widely available commercially.

Recently, efforts have been made to identify peptide fragments of native hirudin which are also effective in lowering clotting time. An unsulfated 21 amino acid C-terminal fragment of hirudin, $N^{\alpha}$-acetylhirudin$_{45-65}$, inhibits clot formation in vitro. In addition, several other smaller, unsulfated peptides corresponding to the C-terminal 11 or 12 amino acids of hirudin (residues 55-65 and 54-65) have also demonstrated efficacy in inhibiting clot formation in vitro [J. L. Krstenansky et al., "Antithrombin Properties of C-terminus of Hirudin Using Synthetic Unsulfated $N^{\alpha}$-acetyl-hirudin$_{45-65}$", FEBS Lett, 211, pp. 10-16 (1987)]. Such peptide fragments, however, may not be fully satisfactory to dissolve blood clots in on-going therapy regimens. For example, $N^{\alpha}$-acetylhirudin$_{45-46}$ has a specific activity four orders of magnitude lower than native hirudin.

In addition to catalyzing the formation of a fibrin clot, thrombin has several other bioregulatory roles [J. W. Fenton, II, "Thrombin Bioregulatory Functions", Adv. Clin. Enzymol., 6, pp. 186-93 (1988)]. For example, thrombin directly activates platelet aggregation and release reactions. This means that thrombin plays a central role in acute platelet-dependent thrombosis [S. R. Hanson and L. A. Harker, "Interruption of Acute Platelet-Dependent Thrombosis by the Synthetic Antithrombin D-Phenylalanyl-L-Prolyl-L-Arginylchloromethylketone", Proc. Natl. Acad. Sci. USA, 85, pp. 3184-88 (1988)].

Thrombin can also directly activate an inflammatory response by stimulating the synthesis of platelet activating factor (PAF) by endothelial cells [S. M. Prescott et al., "Human Endothelial Cells in Culture Produce Platelet-Activating Factor (1-alkyl-2-acetyl-sn-glycero-3-phosphocholine) When Stimulated with Thrombin, Proc. Natl. Acad. Sci. USA, 81, pp. 3534-38 (1984)]. PAF is exposed on the surface of endothelial cells and serves as a ligand for neutrophil adhesion and subsequent degranulation [G. M. Vercolletti et al., "Platelet-Activating Factor Primes Neutrophil Responses to Agonists: Role in Promoting Neutrophil- Mediated Endothelial Damage", Blood, 71, pp. 1100-07 (1988)].

The mechanism by which thrombin activates platelets and endothelial cells involves a receptor and is

effected at a lower concentration than that required for fibrinogen cleavage [J. T. Harmon and G. A. Jamieson, "The Glycocalcin Portion of Glycoprotein Ib Expresses Both High and Moderate Affinity Receptor Sites for Thrombin", J. Biol. Chem., 28, pp. 13224-29 (1986)]. Reagents which block the active site of thrombin, such as hirudin, interrupt the activation of platelets and endothelial cells [C. L. Knupp, "Effect of Thrombin Inhibitors on Thrombin-Induced Release and Aggregation", Thrombosis Res., 49, pp. 23-36 (1988)]. However, there is no evidence that the mere interruption of thrombin-receptor binding is sufficient to inhibit that activation.

Accordingly, despite these developments to date, the need exists for small synthetic peptides, characterized by increased efficacy in the inhibition of clot formation, thrombin-induced platelet activation or endothelial cell activation, which may be produced in commercially feasible quantities.

## DISCLOSURE OF THE INVENTION

The present invention solves the problems referred to above by providing peptides characterized by the biological activity of native hirudin. Specifically, the peptides and analogs of this invention, as well as both compositions and combinations containing them, are effective as anticoagulants which cause an increase in blood clotting time. At low dosages, these peptides inhibit thrombin-induced platelet aggregation and platelet release (hereinafter "platelet activation") and the release of inflammatory substances from endothelial cells (hereinafter "endothelial activation") without significantly increasing clotting time. The relatively small size of these peptides (between about 8 and 26 amino acids) advantageously allows them to be produced synthetically. Thus, they may be produced in extremely high yields and are easily purified, as compared to either native hirudin or its full length recombinant DNA counterpart.

Advantageously, the peptides of the present invention, unlike hirudin, exhibit a saturable effect on clotting time. Thus, the therapeutic and prophylatic uses of these peptides avoid the harmful and potentially fatal consequences of an overdose associated with conventional anticoagulants, such as heparin. And the small size of the peptides of this invention decreases the possibility of an adverse antigenic response in patients treated with them.

As will be appreciated from the disclosure to follow, the peptides, compositions, combinations and methods of this invention are useful in the treatment, prevention or diagnosis of vascular diseases attributed to the undesirable effects of thrombin, as well as in in vitro diagnostic assays.

## BRIEF DESCRIPTION OF THE DRAWINGS

Figure 1 depicts the purification of Sulfo-$Tyr_{63}$hirudin$_{53-64}$ by reverse-phase HPLC.

Figure 2 depicts HPLC chromatograms illustrating the relative efficiency of the sulfation process of this invention (Figure 2c) as compared with conventional sulfation processes (Figures 2a, 2b) for the treatment of large quantities of peptide.

Figure 3 depicts the HPLC chromatographic elution profile of a mixture of Sulfonyl-$Tyr_{63}$hirudin$_{53-64}$ and Sulfo-$Tyr_{63}$hirudin$_{53-64}$.

Figure 4 depicts the anticoagulant activity of hirudin$_{53-64}$ and Sulfo-$Tyr_{63}$hirudin$_{53-64}$ over a range of peptide concentrations.

Figure 5 is a table which displays the covalent structures of hirudin peptides according to this invention, as well as their anticoagulant activity. In this figure, the amino acids are represented by single letter codes as follows:

| | | | | | | | |
|---|---|---|---|---|---|---|---|
| Phe: | F | Leu: | L | Ile: | I | Met: | M |
| Val: | V | Ser: | S | Pro: | P | Thr: | T |
| Ala: | A | Tyr: | Y | His: | H | Gln: | Q |
| Asn: | N | Lys: | K | Asp: | D | Glu: | E |
| Cys: | C | Trp: | W | Arg: | R | Gly: | G |

Figure 6 depicts the inhibition of thrombin times by varying amounts of native hirudin and Sulfo-$Tyr_{63}$hirudin$_{53-64}$.

Figure 7 depicts the effects of varying amounts of Sulfo-$Tyr_{63}$hirudin$_{53-64}$ on bovine thrombin time and human thrombin time.

Figure 8 depicts the effects of varying amounts of heparin alone,Sulfo-$Tyr_{63}$hirudin$_{53-64}$ alone, and a 1:1 combination of heparin and Sulfo-$Tyr_{63}$hirudin$_{53-64}$ on activated partial thromboplastin times.

Figure 9 depicts the in vivo anticoagulant activity of Sulfo-$Tyr_{63}$hirudin$_{53-64}$ in a dose response study in Balb/c mice.

Figure 10 depicts the inhibition of Factor IXa activation of Factor X by Sulfo-$Tyr_{63}$hirudin$_{53-64}$.

Figure 11 depicts an SDS-polyacrylamide gel demonstrating the inhibition of Factor Xa activation of prothrombin by Sulfo-$Tyr_{63}$hirudin$_{53-64}$.

Figures 12A-12C depict the synthesis of hirulog-1, hirulog-2 and hirulog-3, peptidomimetic analogs of the peptides according to this invention.

Figure 13A depicts the synthesis of hirulog-4, a peptidomimetic analog of the peptides according to this invention.

Figures 14A and 14B depict the synthesis of hirulog-5 and hirulog-6, peptidomimetic analogs of the peptides according to this invention.

Figure 15 depicts the synthesis of hirulog-7, a peptidomimetic analog of the peptides according to this invention.

Figure 16 depicts the effects of low doses of Sulfo-$Tyr_{63}$hirudin$_{53-64}$ on thrombin-induced platelet aggregation and clotting time.

Figure 17 depicts the effects of $Arg_{53}$hirudin$_{53-64}$ on ADP- and collagen-induced platelet aggregation.

## DETAILED DESCRIPTION OF THE INVENTION

The present invention relates to peptides which correspond to the amino acid sequence of the carboxyl terminal portion of hirudin and which display the biological activity of native hirudin. More specifically, the peptides of this invention are homologous to at least a portion of the carboxy terminal 26 amino acids of native hirudin. According to one embodiment of this invention, such peptides are derivatized at the single tyrosine residue by the addition of a negatively charged side group.

Hirudin peptides of this invention include, but are not limited to: peptides characterized by a sequence of amino acids consisting substantially of the formula: Asn-Gly-Asp-Phe-Glu-Glu-Ile-Pro-Glu-Glu-Tyr-X, and D-retro forms thereof; wherein X is selected from the group consisting of COOH, Leu and Leu-Gln. Additionally, hirudin peptides according to this invention include those which are characterized by a sequence of amino acids consisting substantially of the formula: Y-Phe-Glu-Glu-Ile-Pro-Glu-Glu-Tyr-Z, and D-retro forms thereof; wherein Y is selected from the group consisting of $NH_2$, an amino protecting group, at least the C-terminal portion of the amino acid sequence: Val-Thr-Gly-Glu-Gly-Thr-Pro-Lys-Pro-Gln-Ser-His-Asn-Asp-Gly-Asp, and at least the C-terminal portion of the amino acid sequence: Val-Thr-Gly-Glu-Gly-Thr-Pro-Asn-Pro-Glu-Ser-His-Asn-Asn-Gly-Asp; Z is selected from the group consisting of COOH, Leu and Leu-Gln; and the tyrosine residue is characterized by the presence of a negatively charged side group. In such peptides, the negatively charged side group may be selected from the group consisting of sulfate, phosphate, carboxylate, sulfonate, phosphonate, carbonate, methyl sulfonate, methyl phosphonate and variants thereof. According to one embodiment of this invention, such peptides include those in which Y is Asn-Gly-Asp and Z is Leu and the tyrosine residue is sulfated. In addition, the peptides of the present invention may be characterized by the presence of an N-acetyl group on the amino terminal amino acid.

The production of the peptides of this invention may be achieved by a variety of methods known in the art. For example, the peptides may be derived from the intact hirudin molecule by proteolysis using specific endopeptidases in combination with exopeptidases, Edman degradation, or both. The intact hirudin molecule, in turn, may be purified from its natural source, H. medicinalis, using conventional methods. Alternatively, hirudin may be produced by known recombinant DNA techniques using cDNAs [R. P. Harvey et al., Proc. Natl. Acad. Sci. USA, 83, pp. 1084-88 (1986); J. Dodt et al., "Expression Secretion and Processing of Hirudin in E. coli Using the Alkaline Phosphatase Signal Sequence", FEBS Lett., 202, pp. 373-77 (1986)], or a chemically synthesized gene [C. Bergmann et al., "Chemical Synthesis and Expression of a Gene Coding for Hirudin, the Thrombin-Specific Inhibitor from the Leech, Hirudo medicinalis", Biol. Chem. Hoppe-Seyler, 367, pp. 731-40 (1986)].

Preferably, the peptides of the present invention are produced directly, thus eliminating the need for an entire hirudin molecule as a starting material. This may be achieved by well known recombinant DNA

techniques wherein only those DNA sequences which encode the desired peptides are expressed in transformed hosts.

Alternatively, the peptides of this invention may be produced by conventional chemical synthesis techniques. In a preferred embodiment of the invention, hirudin peptides are synthesized by solution phase or solid phase peptide synthesis and, optionally, digested with carboxypeptidase (to remove C-terminal amino acids) or degraded by manual Edman degradation (to remove N-terminal amino acids). Peptides produced in this way may then be purified by separation techniques widely known in the art, preferably utilizing reverse phase HPLC. The use of solution phase synthesis advantageously allows for the direct addition of derivatized amino acids to the growing peptide chain. This obviates the need for a subsequent derivatization step to modify the tyrosine residue of the peptides of this invention.

Throughout this specification and in the claims, the abbreviations employed for amino acids and their residues are used in conformity with the generally accepted rules of nomenclature and relate to $\alpha$-amino acids and their residues of the L-series. However, the present invention also relates to the D-retro forms of the peptides described herein. These are produced by synthesis with D amino acids in the opposite orientation, beginning with the carboxy terminal amino acid of the L form.

Derivatization of the hirudin peptides of the invention may involve the addition of a negatively charged side group onto either the free phenolic hydroxyl or the benzoyl meta carbon of the single tyrosine residue. The derivatization may involve the addition of a variety of negatively charged side groups which are known in the art. Derivatization methods include, but are not limited to, sulfation, methyl sulfonation, phosphorylation, methyl phosphonation and carboxylation of the tyrosine hydroxyl group and sulfonation, phosphonation and carbonation of the tyrosine benzoyl meta carbon. Techniques for performing these reactions are also well known in the art.

Most preferably, the peptides of this invention are derivatized by sulfation. A preferred peptide of this invention is Sulfo-$Tyr_{63}$$hirudin_{53-64}$ (subscript numbers represent the corresponding amino acid position in the native hirudin molecule), a 12 amino acid peptide, having a sulfated tyrosine residue, that is homologous to residues 53-64 of native hirudin. Another preferred peptide is Sulfonyl-$Tyr_{63}$$hirudin_{53-64}$ which has a sulfonated tyrosine residue. This sulfonated peptide may possess a longer biological half-life than its sulfated counterpart.

Sulfation of the hirudin peptides of this invention may be achieved either by a biological (enzymatic) or a chemical process. In a preferred embodiment of this invention, a purified peptide of this invention is reacted concurrently with dicyclohexylcarbodiimide and sulfuric acid in an organic solvent. Sulfonation of the meta carbon results as a side reaction of this sulfation process.

For large-scale sulfations, the sulfation procedure is modified, so that gram quantities of the peptide are first dissolved in an organic solvent, preferably dimethylformamide, and then reacted with a dehydrating agent, preferably dicyclohexylcarbodiimide. The dehydrated tyrosine residue of the peptide is then sulfated by reaction with sulfuric acid. The reaction is complete upon formation of an insoluble dicyclohexyl urea salt. This modification results in high yields of sulfated peptide on a large scale. This novel sulfation technique may be used to sulfate the tyrosine residues of any peptide or polypeptide whether isolated and purified or present in a crude preparation. Following either sulfation reaction, the sulfated peptide may be separated from any sulfonated peptide, as well as from unreacted peptide by HPLC, DEAE chromatography, or any of several other conventional separation techniques.

Sulfation may also be achieved by reacting a peptide of this invention with sulfur trioxidetriethylamine salt in pyridine. In addition, a tyrosylsulfotransferase activity, either as a crude preparation or as a purified enzyme, may be used to sulfate the tyrosine residue [R. W. H. Lee and W. B. Huttner, "Tyrosine O Sulfated Proteins of PC-12 Pheo Chromo Cytoma Cells and Their Sulfation By a Tyrosyl Protein Sulfotransferase", J. Biol. Chem., 258, pp. 11326-34 (1983)]. Phosphorylation or carboxylation of the hirudin peptides of this invention may be achieved by reactions similar to those described above for sulfation, with the substitution of phosphoric acid or formic acid, respectively, for sulfuric acid. In those reactions, phosphonation or carbonation will occur, respectively, as a side reaction. Alternatively, enzymatic methods may be employed for carboxylation or phosphorylation of the hirudin peptides of this invention.

Methyl sulfonation and methyl phosphonation of the hirudin peptides of this invention may be achieved by methods well-known in the art including, but not limited to, alkylation with chlorosulfonic or chlorophosphonic acid, respectively.

The extent of the sulfation reaction may be followed spectrophotometrically. The absorbance spectra of sulfated peptides reveal a shift in maximal absorbance from approximately 275 nm to approximately 250-265 nm. Confirmation of derivatization may be obtained by desulfating the peptide with 30% trifluoroacetic acid at 60° C for 30 minutes. This will result in an increase of maximal absorbance back to 275 nm.

According to an alternate embodiment of this invention, hirudin peptides may also be derivatized at

their amino terminus by the addition of an N-acetyl group. N-acetylation may be achieved by any of a number of techniques that are known to those of skill in the art. Preferably, acetylation is achieved by using an N-acetyl amino acid derivative in the synthesis of the peptides of this invention. Alternatively, N-acetylation may be achieved by reacting the peptide with acetic anhydride. The N-acetylated hirudin peptides of this invention advantageously demonstrate increased biostability as compared to their corresponding unacetylated peptide counterparts.

The anticoagulant activity of the hirudin peptides of this invention may be assayed using any conventional technique. Preferably, the assay involves direct determination of the thrombin-inhibitory activity of the peptide. Such assays measure the inhibition of thrombin-catalyzed cleavage of colorimetric substrates or, more preferably, the increase in activated partial thromboplastin times (APTT) and increase in thrombin times (TT). The latter assays measure factors in the "intrinsic" pathway of coagulation. Alternatively, the assay employed may use purified thrombin and fibrinogen and measures the inhibition of release of fibrinopeptides A or B by radioimmunoassay or ELISA.

The anticoagulant potency of the peptides of the present invention depends, in part, on their in vivo half-life. Accordingly, this invention also relates to pharmaceutical compositions, either covalent or non-covalent, comprising hirudin peptides coupled to pharmaceutically acceptable polymers which increase the biological half-life of those peptides. For example, a hirudin peptide of this invention may be coupled to an activated derivative of polyethyleneglycol (PEG) using conventional techniques. Preferably, a PEG N-succinimidyl succinate is attached to the α-amino moiety of the peptide. Such attachment is effected by reacting the peptide with the PEG N-succinimidyl succinate reagent (SS-PEG) in an organic solvent or a buffered solution having a pH greater than about 7.0. Most preferably, about a 50-fold molar excess of SS-PEG (Avg. MW = 5,000 daltons) is reacted with a peptide in a 20 mM sodium borate buffer, pH 9.0.

The hirudin peptides of this invention are useful alone, or in compositions, combinations and methods for the treatment and prophylaxis of vascular diseases attributed to blood system thromboses. For example, the peptides of this invention, and compositions and combinations containing them, may be used for heparin replacement for prophylactic purposes, heparin replacement in the treatment of thrombocytopenia, treatment of disseminated intravascular coagulation and treatment of vascular thrombi that may arise from any disease state. The hirudin peptides of this invention, as well as compositions and combinations containing them, may be used in the treatment or prophylaxis of vascular diseases in patients including mammals and, in particular, humans.

Alternatively, the hirudin peptides of this invention may be administered in combination with heparin or low molecular weight heparin. Such combinations advantageously lower the dosage of heparin or low molecular weight heparin required to produce a desired anticoagulant effect when either compound is used alone. Furthermore, these combinations advantageously reduce the potential for hemorrhagic complications often associated with heparin use. And such combinations surprisingly demonstrate greater anticoagulant activity than that exhibited in monotherapies based upon either of the individual components. As defined herein, the term "combination" includes a single dosage form containing at least one peptide of the present invention and heparin or low molecular weight heparin, a multiple dosage form wherein the two agents are administered separately, but concurrently, or a multiple dosage form wherein the two agents are administered separately, but sequentially.

The hirudin peptides of this invention may be formulated using conventional methods to prepare pharmaceutically useful compositions and combinations. Such compositions preferably include at least one pharmaceutically acceptable carrier. See, e.g., Remington's Pharmaceutical Sciences (E. W. Martin). Pharmaceutical compositions of the present invention typically contain, in addition to the hirudin peptides, a pharmaceutically acceptable buffer, preferably phosphate buffered saline, together with a pharmaceutically acceptable compound for adjusting isotonic pressure, such as sodium chloride, mannitol or sorbitol. Compositions of the present invention may also contain, in addition to the above components, a low dosage of heparin or low molecular weight heparin, preferably less than about 2,500 to 5,000 units.

Various dosage forms may be employed to administer the hirudin peptide-containing compositions and combinations of this invention. These include, but are not limited to, parenteral administration, oral administration and topical application. The dosage and dose rate will depend on a variety of factors such as the specific composition, the object of the treatment, i.e., therapy or prophylaxis, and the judgment of the treating physician.

Compositions for injection may be in the form of lyophilizates or solutions. Compositions formulated for topical application may, for example, be in aqueous jelly, oily suspension or emulsified ointment form. The resulting formulations will contain an amount of hirudin peptide effective to prevent or reduce blood clots.

For administration by injection, a therapeutic amount of the peptides of this invention will normally be in the daily dosage range of about 0.2 to 75 mg/kg body weight, preferably in the range of about 0.5 to 10

mg/kg body weight. Methods for determining effective dosages are known to those skilled in the art. However, those dosages are approximately equal to or less than those currently used with other thrombolytic agents, e.g., about 3.5 mg/kg body weight, followed by a maintenance dose of 7 mg/hr i.v. for about 7 to 10 days. Because the PEG derivatized hirudin peptides of this invention display a longer half-life as compared to native hirudin, as well as the underivatized peptides, their dosage amounts are advantageously well below those specified above for the underivatized peptides.

The compositions and combinations of this invention may additionally contain other components which are effective in fibrinolytic therapy. These include, but are not limited to, tissue plasminogen activator, urokinase and streptokinase. These other compounds may be present as separate components in the hirudin peptide-containing compositions of this invention. Alternatively, the hirudin peptides may be conjugated to such fibrinolytic agents. Conjugation may be achieved by any of the methods known in the art. The hirudin peptides and fibrinolytic agents may also be present as a single molecule, i.e., in the form of a fusion protein, produced by recombinant DNA techniques or by in vitro synthesis.

This invention also relates to compositions containing the hirudin peptides of this invention and methods of using such compositions for the treatment of tumor metastases. The efficacy of the hirudin peptides of this invention for the treatment of tumor metastases via inhibition of metastatic growth is based upon the presence of a procoagulant enzyme present in certain cancer cells [A. Falanga and S. G. Gordon, "Isolation and Characterization of Cancer Procoagulant: A Cysteine Proteinase from Malignant Tissue", Biochemistry, 24, pp. 5558-67 (1985); S. G. Gordon et al., "Cysteine Proteinase Procoagulant from Amnion-Chorion", Blood, 66, pp. 1261-65 (1985); and A. Falanga et al., "A New Procoagulant In Acute Leukemia", Blood, 71, pp. 870-75 (1988)]. This enzyme activates the conversion of Factor X to Factor Xa in the coagulation cascade, resulting in fibrin deposition which, in turn, serves as a substrate for tumor growth. Therefore, by inhibiting fibrin deposition through the inhibition of Factor Xa, thrombin or both, the hirudin peptides of the present invention serve as effective anti-metastatic tumor agents. Examples of metastatic tumors which may be treated by the peptides of this invention include, but are not limited to, carcinoma of the brain, carcinoma of the liver, carcinoma of the lung, osteocarcinoma and neoplastic plasma cell carcinoma.

This invention also relates to methods and compositions employing low dosages of the peptides and analogs of this invention to inhibit thrombin-induced platelet activation and thrombin-induced endothelial cell activation. The inhibition of endothelial cell activation effected by the methods and compositions of this invention includes the repression of platelet activating factor (PAF) synthesis by these cells. This mechanism of inhibition has important implications in the treatment of diseases characterized by thrombin-induced inflammation, which is thought to be mediated by PAF. The present invention also relates to compositions comprising low dosages of the peptides and analogs of this invention and methods which employ them for the treatment of patients suffering from diseases characterized by thrombin-induced inflammation. Such diseases include, but are not limited to, adult respiratory distress syndrome, septic shock, septicemia and reperfusion damage.

The use of low dosages of hirudin peptides surprisingly and unexpectedly permits the above-described inhibitory activities to be achieved in a treated patient, with minimal anticoagulant effects. Accordingly, the use of low dosages of hirudin peptides is advantageous in circumstances where inhibition of activation of platelets and endothelial cells or treatment of diseases characterized by thrombin-induced inflammation are desirable without concomitant anticoagulant effects.

Preferred compositions for inhibiting platelet and endothelial cell activation according to this invention comprise between about 0.0001 mg/kg body weight and about 0.099 mg/kg body weight. More preferably, these compositions comprise between about 0.001 mg/kg body weight and about 0.05 mg/kg body weight.

This invention also relates to the use of hirudin peptides, or compositions containing them, as anticoagulants for extracorporeal blood. As used in this application, the term "extracorporeal blood" includes blood removed in line from a patient, subjected to extracorporeal treatment, and then returned to the patient in processes such as dialysis procedures or blood filtration or blood bypass during surgery. The term also includes blood produots which are stored extracorporeally for eventual administration to a patient. Such products include whole blood, plasma or any blood fraction in which inhibition of coagulation is desired. Such compositions are similar to the above-described injectable preparations. The amount or concentration of active peptide in these types of compositions will be based on the volume of blood to be treated or, more preferably, its thrombin content.

This invention also relates to the bioanalytic use of hirudin peptides, or compositions containing them, for determining the concentration of Factor IXa, Factor Xa, thrombin, or mixtures thereof, in a biological sample. These peptides and compositions may be used in a manner similar to that of reagents employed in conventional assays. In addition, the peptides of this invention may be utilized in presently available methods and kits designed to detect Factor IXa, Factor Xa, thrombin, or mixtures thereof.

Furthermore, the peptides of the present invention may be used for ex vivo thrombus imaging in humans and other mammals. In one embodiment of this invention, the hirudin peptides are radiolabeled with a radioisotope. The choice of radioisotope is based upon a number of well-known factors, for example, toxicity, biological half-life and detectability. Preferred radioisotopes include, but are not limited to, $^{125}$I, $^{123}$I and $^{111}$In. Techniques for labeling peptides are well known in the art. Most preferably, the radioisotope is $^{123}$I and labeling is achieved using $^{123}$I-Bolton Hunter Reagent. The labeled peptide is administered to a patient, preferably by an intravenous route, and allowed to bind to the thrombin contained in a fibrin clot. The clot is then observed by utilizing well-known detecting means, such as a camera capable of detecting radioactivity coupled to a computer imaging system. This technique also yields images of platelet-bound thrombin and meizothrombin.

This invention also relates to peptidomimetic analogs of the hirudin peptides described herein. Peptidomimetic analogs mimic the three-dimensional structure of the active site contained in the parent peptide. We believe these analogs display similar activities as their peptide counterparts. According to one embodiment of the present invention, the analogs of the hirudin peptides may be either semi-peptidic or non-peptidic in nature. These peptidomimetic analogs advantageously exhibit increased shelf-life and biostability when compared to the parent compound. Moreover, the bioavailability of the peptidomimetic analogs of the present invention may be greater than the corresponding peptides when administered by oral or topical routes. Furthermore, these analogs may exhibit increased anticoagulant activity.

This invention also relates to analogs of the hirudin peptides of this invention which are capable of forming covalent bonds to thrombin. According to one embodiment, these analogs are characterized by the presence of a dinitrofluorobenzyl group attached to the amino terminus of the peptides. According to another embodiment, these analogs are characterized by the replacement of tyrosine or derivatized tyrosine, as well as any other more caboxy terminal residues, with nitroanisole. Both classes of analogs possess the ability to form a covalent bond with thrombin and therefore have greater affinity for that molecule. As a result of this greater affinity, these hirudin peptide analogs may be substantially more potent than the other hirudin peptides of this invention which bind to thrombin via ionic interactions.

It should be understood that the peptidomimetic and covalent analogs of this invention are characterized by biological activities that are similar to those of the hirudin peptides described herein. Accordingly, these analogs may be employed in compositions, combinations, and methods for diagnosis, therapy and prophylaxis in the same manner as the peptides of this invention.

This invention also relates to hirudin peptides which are identical to the above-described peptides, except they are characterized by the replacement of $Asp_{53}$ or $Asn_{53}$ with an arginine residue. These peptides contain an $Arg_{53}$-$Gly_{54}$-$Asp_{55}$ sequence which binds to and inhibits the platelet surface glycoprotein IIb/IIIa. These peptides surprisingly and unexpectedly display both inhibitory acitivity against platelet activation induced by any agonist, as well as anticoagulant activity. Moreover, the presence of the Arg-Gly-Asp sequence serves to target these peptides to the site of a platelet-rich clot. Once the peptides reach this target, they inhibit both additional platelet aggregation and the generation of fibrin. This action prevents the expansion of a blood clot, effectively resulting in increased clot dissolution. These novel peptides may be employed in compositions and methods for achieving the dual effects of increasing clotting time and inhibiting platelet activation.

According to another embodiment of the present invention, one or a combination of any of the hirudin peptides or analogs may be used in compositions and methods for coating invasive devices to be inserted into a patient. These compostions and methods result in lower risk of thrombotic complications in patients receiving such devices. Surfaces that may be coated according to the methods and compositons of this invention are exemplified by those of prostheses, artificial valves, vascular grafts, stents and catheters. Methods for achieving the coating of these surfaces are known in the art. These include chemical cross-linking or physical adsorption of the hirudin peptide- or analog-containing compositions to the surfaces of the devices.

In order that this invention may be more fully understood, the following examples are set forth. It should be understood that these examples are for illustrative purposes only and are not to be construed as limiting this invention in any manner.

In all of the examples of peptide syntheses described below, we carried out amino acid analysis of the synthesized peptides. Amino acid hydrosylates were prepared by treatment of samples in 6 N hydrochloric acid, in vacuo, at 110 °C for 24 hours, followed by ion-exchange chromatography employing a Beckman System 6300 analyzer.

We routinely analyzed purity of the synthetic peptides by reverse-phase HPLC. Unless otherwise specified, peptide samples (20-100 µg) were applied to a Vydac $C_4$ column (0.46 x 25 cm) or an Aquapore RP-300 $C_8$ column (0.46 x 3.0 cm) using a Beckman Liquid Chromatographic System or an Applied

Biosystems 150A Chromatographic System, respectively. The Vydac $C_4$ column was equilibrated in water containing 0.1% trifluoroacetic acid (TFA) and developed with a gradient of increasing acetonitrile concentration from 0 to 80% in the same TFA-containing solvent. The gradient was developed over 30 minutes at a flow rate of 1.0 ml/min. The effluent stream was monitored at 215 nm for absorbance. The Aquapore $C_8$ column was equilibrated in water containing 0.1% TFA and developed with an increasing gradient of acetonitrile concentration from 0 to 70% in a 0.085 % TFA solvent. The gradient was developed for 45 minutes at a flow rate of 0.5 ml/min. The effluent stream was then monitored at 214 nm for absorbance.

## EXAMPLE 1

### Synthesis Of Hirudin$_{53-64}$ And Hirudin$_{49-64}$

Hirudin$_{53-64}$ has the amino acid formula:
$H_2$N-Asn-Gly-Asp-Phe-Glu-Glu-Ile-Pro-Glu-Glu-Tyr-Leu-COOH. Hirudin$_{49-64}$ has the amino acid formula:
$H_2$N-Glu-Ser-His-Asn-Asn-Gly-Asp-Phe-Glu-Glu-Ile-Pro-Glu-Glu-Tyr-Leu-COOH. We prepared these peptides as part of a single synthesis by solid-phase peptide synthesis employing an Applied Biosystems 430 A Peptide Synthesizer (Applied Biosystems, Foster City, Ca.).

Specifically, we reacted 0.259 meq of Boc-Leu-O-resin (1% divinylbenzene resin (DVB)) sequentially with 2 mmoles of protected amino acids. Following 11 cycles of synthesis, 0.42 g of wet resin were removed from the reaction vessel. The remaining 0.43 g aliquot of wet resin was reacted with two times 2 mmoles of protected amino acids for four cycles. Hirudin$_{53-64}$ and hirudin$_{49-64}$ thus synthesized were fully deprotected and cleaved from the resin by treatment with anhydrous HF: p-cresol: ethyl methyl sulfate (10:1:1, v/v/v). Yield of the peptides was 56% and 53% for hirudin$_{49-64}$ and hirudin$_{53-64}$, respectively.

Individual HPLC analysis of the peptides revealed a high degree of purity and single predominant peaks of 214 nm-absorbing material eluting at 16.1 min and 16.3 min, respectively, for hirudin$_{49-64}$ and hirudin$_{53-64}$.

## EXAMPLE 2

### Synthesis Of des(Tyr-Leu) Hirudin$_{53-62}$

Des(Tyr-Leu) hirudin53-62 has the amino acid formula: $H_2$N-Asn-Gly-Asp-Phe-Glu-Glu-Ile-Pro-Glu-Glu-COOH. We prepared des(Tyr-Leu) hirudin$_{53-62}$ as follows:

First, we prepared carboxypeptidase A, essentially as described by R. P. Ambler, "Enzymatic Hydrolysis with Carboxypeptidases", Methods Enzymol., 25, part B, pp. 143-54. The enzyme (1.0 mg) was suspended in 1.0 ml of deionized water at 4° C and centrifuged in a microfuge apparatus. We discarded the supernatant and added 100 µl of 1% sodium bicarbonate to the precipitate. We then added 0.1N NaOH dropwise until the precipitate dissolved. The pH was then adjusted to 8.0 by the dropwise addition of 0.1N HCl. We adjusted the concentration of the enzyme to 1 mg/ml by the addition of 0.1M N-ethylmorpholine acetate, pH 8.25.

We dissolved 1.3 mg of hirudin$_{53-64}$, as prepared in Example 1, in 250 µl of N-ethylmorpholine acetate, pH 8.25, containing 1.0 M NaCl. We then added 30 µl (30 µg) of carboxypeptidase A as prepared above and incubated the reaction for 2 hours at 37° C.

Peptide fragments were purified by reverse phase HPLC employing an Aquapore RP-300 $C_8$ column (0.46 x 3.0 cm) and an Applied Biosystems 150 A liquid chromatographic system. The column was equilibrated in water containing 0.1% TFA and developed with a gradient of increasing acetonitrile concentration from 0 to 35% over 45 minutes at a flow rate of 0.5 ml/min in a 0.085% TFA-containing solvent. The effluent stream was monitored for absorbance at 214 nm. Fractions were collected manually, dried under vacuum and analyzed for amino acid composition and effects on clotting time of human plasma. We observed that des(Tyr-Leu) hirudin$_{53-62}$ elutes prior to any remaining intact hirudin$_{53-64}$.

## EXAMPLE 3

### Synthesis Of $Hirudin_{57-64}$

$Hirudin_{57-64}$ has the amino acid formula:
$H_2N$-Glu-Glu-Ile-Phe-Glu-Glu-Tyr-Leu-COOH. To synthesize $hirudin_{57-64}$, we followed the procedure outlined in Example 1, except that we used 0.55 mmoles of Boc-Leu-$OCH_2$-PAM resin (1% DVB) (Applied Biosystems) in place of the Boc-Leu-O-resin (1% DVB). We then added 2 mmoles of protected amino acids at each cycle of coupling. The yield of crude peptide was 17.9%. HPLC analysis revealed a single predominant peak eluting at 14.2 min in the gradient.

## EXAMPLE 4

### Synthesis Of $Hirudin_{45-64}$

$Hirudin_{45-64}$ has the amino acid formula:
$H_2N$-Thr-Pro-Asn-Pro-Glu-Ser-His-Asn-Asn-Gly-Asp-Phe-Glu-Glu-Ile-Pro-Glu-Glu-Tyr-Leu-COOH. We synthesized $hirudin_{45-64}$ using 0.259 meq of Boc-Leu-O-resin (1% DVB). We followed the procedure outlined in Example 1, except that we used 2 mmoles of protected amino acids in each coupling step for the first 13 cycles of synthesis. For the remaining 6 cycles of synthesis, we used two times 2 mmoles of protected amino acids. The peptide was fully deprotected and uncoupled from the DVB resin by treatment with anhydrous HF: p-cresol: ethyl methyl sulfate (10:1:1, v/v/v). Approximately 100 mg of peptide was recovered by extraction of the resin with 30% acetic acid. The yield of $hirudin_{45-64}$ was 17%.

A HPLC analysis revealed a high degree of purity (>90%) in the product and a single predominant peak of 214 nm absorbing material at 14.4 min in the acetonitrile gradient.

## EXAMPLE 5

### Synthesis Of $Hirudin_{55-64}$

$Hirudin_{55-64}$ has the amino acid formula:
$H_2N$-Asp-Phe-Glu-Glu-Ile-Pro-Glu-Glu-Tyr-Leu-COOH. $Hirudin_{55-64}$ was prepared by the procedure outlined in Example 1, using 0.0259 meq of Boc-Leu-O-resin (1% DVB). We then added 2 mmoles of protected amino acids to the growing peptide at each cycle of coupling. Deprotection and cleavage from the resin was achieved as in the previous examples. The recovery of this peptide was 30%.

HPLC analysis revealed a high degree of purity (>95%) in the sample and a single predominant peak at 16.1 min in the acetonitrile gradient.

## EXAMPLE 6

### Synthesis Of $Hirudin_{64-45}$

$Hirudin_{64-45}$ has the amino acid formula:

$H_2N$-Leu-Tyr-Glu-Glu-Pro-Ile-Glu-Glu-Phe-Asp-Gly-Asn-Asn-His-Ser-Glu-Pro-Asn-Pro-Thr-COOH. We synthesized hirudin$_{64-45}$ by the procedure outlined in Example 1, except that we used 0.259 meq of Boc-O-benzyl-L-Thr-O-resin (1% DVB) in place of the Boc-Leu-O-resin (1% DVB). We used 2 mmoles of protected amino acids in each coupling step for the first 6 cycles of synthesis. For the remaining cycles of synthesis, we used two times 2 mmoles of protected amino acids. The peptide was fully deprotected and uncoupled from the DVB resin by treatment with anhydrous HF. Following extraction with 30% acetic acid, 120 mg of peptide was recovered and the yield of hirudin$_{64-45}$ was 19.9%.

HPLC analysis of the peptide revealed a high degree of purity in the preparation (>90%) and a single predominant peak eluting at 13.7 min in the acetonitrile gradient.

We used hirudin$_{64-45}$ as a control in measuring the anticoagulant activities of various peptides of this invention.

## EXAMPLE 7

### Synthesis Of Hirudin$_{54-64}$

Hirudin$_{54-64}$ has the amino acid formula:
$H_2N$-Gly-Asp-Phe-Glu-Glu-Ile-Pro-Glu-Glu-Tyr-Leu-COOH. We synthesized hirudin$_{54-64}$ by the same procedure outlined in Example 1. We used 2 mmoles of protected amino acids in each coupling cycle of synthesis. After synthesis, the peptide was fully deprotected and uncoupled from the DVB resin as in the previous examples.

HPLC analysis revealed a high degree of purity (>60%) in the product and a single predominant peak of 214 nm absorbing material.

## EXAMPLE 8

### Further Purification Of Hirudin Peptides By HPLC

For the purposes of activity analyses, hirudin$_{45-64}$, hirudin$_{49-64}$ and hirudin$_{53-64}$, as prepared above, were purified to homogeneity by preparative reverse-phase HPLC employing a Waters Associates (Milford, MA) liquid chromatography system. Samples of the crude peptides (25 mg each of hirudin$_{45-64}$ and hirudin$_{49-64}$, 30 mg of hirudin$_{53-64}$) were dissolved in 2.0 ml of 0.1% TFA in water. An additional 1.0 ml of 6 M guanidinium chloride was added to crude samples of hirudin$_{49-64}$ and hirudin$_{53-64}$ to increase solubility. The samples were separately injected on a Vydac $C_{18}$ column (22 mm x 25 cm) previously equilibrated in 0.1% TFA in water. The column was developed with a linear gradient of increasing acetonitrile concentration from 0 to 80% over 45 minutes in the same TFA-containing solvent at a flow rate of 4.0 ml/min. The effluent stream was monitored at 229 nm and fractions collected manually.

Other hirudin peptides according to this invention may be similarly prepared and purified.

## EXAMPLE 9

### Synthesis Of N-Acetyl Hirudin$_{53-64}$

N-acetylation of the hirudin peptides of this invention was achieved directly during peptide synthesis. For example, N-acetyl hirudin$_{53-64}$ was synthesized by the basic procedure used to synthesize hirudin$_{53-64}$ described in Example 1. In order to carry out N-acetylation, however, we modified the

procedure by substituting 2 mmoles of N-acetyl-asparagine for 2 mmoles of asparagine in the final cycle of peptide synthesis. Other peptides of the present invention may be similarly N-acetylated by substituting the N-acetyl form of the amino terminal amino acid for the unacetylated form in the last cycle of peptide synthesis.

## EXAMPLE 10

### Sulfation Of Hirudin Peptides

Hirudin$_{53-64}$ was O-sulfated at the tyrosine residue to prepare Sulfo-Tyr$_{63}$hirudin$_{53-64}$, using the chemical modification procedure of T. Nakahara et al., "Preparation of Tyrosine-O-[$^{35}$S] Sulfated Cholecystokinin Octapeptide from a Non-Sulfated Precursor Peptide", Anal. Biochem., 154, pp. 194-99 (1986). We dissolved 1.5 mg of hirudin$_{53-64}$, as prepared in Example 9, in 50 µl of dimethylformamide and dried the solution under $N_2$. The peptide was then redissolved in 40 µl of dimethylformamide (DMF) containing 2 x $10^{-5}$ moles of sulfuric acid. To this we added 10 µl of a solution containing 50 µg N,N′-dicyclohexylcarbodiimide in 40 µl DMF (7.0 x $10^{-5}$ moles). The reaction was allowed to proceed for about 5-10 min at 25° C before the addition of 750 µl of deionized water. Any insoluble reaction products were removed by centrifugation in a microfuge apparatus prior to further purification.

Sulfo-Tyr$_{63}$hirudin$_{53-64}$ was purified away from other peptide and reaction components by reverse-phase HPLC employing a Vydac $C_{18}$ column (4.6 x 25 cm) and an Applied Biosystems, Inc., liquid chromatographic system. The column was equilibrated in a 0.1% TFA-water solvent and developed with a linear gradient of increasing acetonitrile concentration from 0 to 35% over 90 min at a flow rate of 0.8 ml/min with a 0.085% TFA-containing solvent. Fractions were collected, dried in a speed-vac apparatus and redissolved in deionized water. As shown in Figure 1, a large number of peaks of 214 nm absorbing material were resolved.

By assaying the peak fractions for anticoagulant activity, we identified two potential Sulfo-Tyr$_{63}$hirudin$_{53-64}$-containing fractions (peaks A and B; Figure 1). Ultraviolet spectral analysis of peak A at neutral pH revealed a maximal absorbance at 258-264 nm, indicating the presence of a modified tyrosine residue. Amino acid analysis of the peptide in peak A confirmed the hirudin$_{53-64}$ structure. These data demonstrated that peak A contained Sulfo-Tyr$_{63}$hirudin$_{53-64}$.

We confirmed the presence of Sulfo-Tyr$_{63}$ hirudin$_{53-64}$ by treating the peptide of peak A with 30% TFA at 60° C for 1 hour to remove the sulfate group. We then dried the peptide, redissolved it in water and subjected it to reverse-phase HPLC. We carried out HPLC analysis of the desulfated Sulfo-Tyr$_{63}$hirudin$_{53-64}$ using an Aquapore RP-300 C8 column (0.46 x 3.0 cm) and an Applied Biosystems 150A HPLC system. The column was equilibrated in water containing 0.1% TFA and developed with a gradient of increasing acetonitrile concentration from 0 to 70% over 45 minutes at a flow rate of 0.5 ml/min in a 0.085% TFA-containing solvent. The peptide showed HPLC chromatographic behavior identical to that of unsulfated hirudin$_{53-64}$. In addition, peak absorbance of the treated peptide returned to 275-280 nm, typical for a peptide containing an unmodified tyrosine residue.

We then applied the above-described sulfation procedure to large quantities of the corresponding N-acetylated peptide. As shown in the HPLC chromatogram of Figure 2a, treatment of 25 mg of N-acetyl-hirudin$_{53-64}$ (as prepared in Example 9) by the Nakahara procedure produced an 80.1% yield of the desired Tyr-sulfated product. However, efforts to scale the reaction proportionally to 50 mg of N-acetyl-hirudin$_{53-64}$ only resulted in a 48.5% yield of the Tyr-sulfated derivative (Fig. 2b).

Accordingly, we significantly modified the chemistry of the Nakahara procedure to achieve high yield of the Tyr-sulfated derivative in a large scale sulfation reaction. More specifically, we dissolved 1 g of N-acetyl-hirudin$_{53-64}$ in 40 ml of dimethylformamide in the presence of 5.0 ml of N,N′-dicyclohexylcarbodiimide (0.2 g/0.16 ml dimethyl formamide). The mixture was stirred at 0° C and 0.5 ml of concentrated sulfuric acid was added dropwise to the reaction mixture until a precipitate formed. Following 5 minutes, the reaction was stopped by the addition of 40 ml water. Reverse-phase HPLC separation of the reaction mixture (Fig. 2c) indicated a 81.7% yield of the sulfated peptide, Sulfo-Tyr$_{63}$-N-acetyl-hirudin$_{53-64}$ .

Large-scale purification of the sulfated hirudin peptide was then achieved by a one-step anion exchange chromatography. Specifically, crude Sulfo-Tyr$_{63}$-N-acetyl-hirudin$_{53-64}$ was purified on a column of DEAE-Sepharose (250 ml wet resin/5 g crude peptide). The column was pre-equilibrated and the sample was

loaded in 20 mM sodium acetate, pH 5.0. The column was developed with a linear NaCl gradient (0-0.4 M). The Sulfo-$Tyr_{63}$-N-acetyl-$hirudin_{53-64}$ eluted at approximately 0.2-0.3 M NaCl, after the unsulfated peptide, but prior to the sulfonated side product Sulfo-$Tyr_{63}$-N-acetyl-$hirudin_{53-64}$ .

Other hirudin peptides of this invention may be sulfated, purified and analyzed by procedures identical to those set forth above.

## EXAMPLE 11

### Sulfonation Of Hirudin Peptides

N-acetyl-$hirudin_{53-64}$, was modified to its Tyr-sulfonated derivative, Sulfonyl-$Tyr_{63}$-N-acetyl-$hirudin_{53-64}$, during the preparation of Sulfo-$Tyr_{63}$-N-acetyl-$hirudin_{53-64}$ as described in Example 10. Sulfonyl-$Tyr_{63}$-N-acetyl-$hirudin_{53-64}$ was a side reaction product obtained during the large-scale sulfation reaction described in that example. Accordingly, Sulfonyl-$Tyr_{63}$-N-acetyl-$hirudin_{53-64}$ was obtained at between 30 to 40% yield and was found to elute prior to Sulfo-$Tyr_{63}$-$hirudin_{53-63}$ in reverse-phase HPLC separations (see Figure 3).

## EXAMPLE 12

### In Vitro Assays Of Anticoagulant Activity

This example illustrates the inhibitory activity of the hirudin peptides of this invention towards thrombin, as measured by the inhibition of activated partial thromboplatin times (APTT). Anticoagulant activity was determined by assaying APTT of pooled, normal human plasma (George King Biomedical, Inc., Overland Park, Kansas) (diluted 4:1, v/v, plasma:water) with a Coag-A-Mate 2001 instrument (General Diagnostics, Inc.. Morris Plains, New Jersey). Specifically, diluted plasma was mixed with stock solutions of hirudin peptides at concentrations ranging from 750 µg/ml in water, for non-sulfated hirudin peptides, to 35 µg/ml in water for sulfated hirudin peptides. The plasma and hirudin peptides were mixed to a final volume of 125 µl prior to each APTT determination.

We began the APTT assay by adding 92 µl of a crude thromboplastin suspension (General Diagnostics) and 100 µl of 0.3 M calcium chloride reagent to each well of a Coag-A-Mate dish (General Diagnostics) per APTT determination, to a final volume of assay mixture per well of 317 µl. The calcium chloride was prepared fresh prior to each assay. Activation times were constant at 180 sec. The results of such an assay comparing the APTT of $hirudin_{53-64}$ and Sulfo-$Tyr_{63}$$hirudin_{53-64}$ are shown in Figure 4. Figure 4 demonstrates the the sulfated hirudin peptide advantageously exhibited a higher level of activity in increasing clotting time than the corresponding unsulfated peptide. The sulfonated peptide, Sulfonyl-$Tyr_{63}$$hirudin_{53-64}$, showed a similar APTT as that observed for Sulfo-$Tyr_{63}$$hirudin_{53-64}$.

Figure 5 shows a comparison of the $MCT_{50}$ (the nmoles of peptide per 125 µl of diluted plasma required to increase clotting time to 50% of a maximal net increase observed in the APTT assay) of various hirudin peptides of this invention. $Hirudin_{53-64}$ demonstrated an $MCT_{50}$ of 0.77. The sulfated counterpart of this peptide, Sulfo-$Tyr_{63}$$hirudin_{53-64}$ advantageously caused an almost 10-fold decrease in $MCT_{50}$. An unsulfated peptide which lacked the asparagine residue ($Asn_{53}$), $hirudin_{57-64}$, had no effect on increasing clotting time when compared to negative control peptide $hirudin_{64-45}$. Similarly, a peptide which did not contain the tyrosine residue, des(Tyr-Leu) $hirudin_{53-62}$, exhibited no comparative increase in clotting time. The presence of an asparagine residue (corresponding to $Asn_{53}$ of the published HV-2 sequence) and a tyrosine residue (corresponding to $Tyr_{63}$ of the hirudin sequence) are believed to be important for the anticoagulant activity of the peptides of this invention.

All of the hirudin peptides which caused a decrease in $MCT_{50}$ did so in a biphasic dose-dependent manner where first, a narrow range of peptide concentration yielded a 20 sec increase in APTT and a second, wider range of peptide concentration resulted in only an additional 10-15 sec increase in clotting

time.

In order to analyze the specificity of the hirudin peptides of this invention towards thrombin, we studied their effect on the increase in thrombin times (TT). Thrombin times were measured using the tilt-tube method. Specifically, we dissolved human α-thrombin (Diagnostica Stago, Asniere, France) in 0.01 M Tris-HCl, pH 7.4, containing 0.15 M NaCl and 10 mM $CaCl_2$ at a concentration of 2.5 U/ml. We mixed 100 µl of the thrombin solution with 200 µl of normal human plasma (George King Biomedical, Inc., KA) that had been premixed with 0 - 10 µg/ml of Sulfo-$Tyr_{63}$$hirudin_{53-64}$ (0.8 U/ml final concentration of thrombin). The thrombin time of normal plasma was typically 15 seconds.

Figure 6 demonstrates a comparison of the increase in thrombin times by hirudin (Sigma St. Louis, MO) and Sulfo-$Tyr_{63}$$hirudin_{53-64}$. A concentration of 3.3 x $10^{-8}$M Sulfo-$Tyr_{63}$$hirudin_{53-64}$ increased TT from 15 to 55 sec. By comparison to hirudin, Sulfo-$Tyr_{63}$$hirudin_{53-64}$ exhibited an approximately 50-fold greater molar specific inhibitory activity towards thrombin. These are the same relative inhibitory activities observed in the APTT assay.

As we have demonstrated above, maximum anticoagulant potency of the unsulfated peptide assayed was exhibited by $hirudin_{53-64}$. A minimum two-fold decrease in inhibitory activity resulted upon removal of the asparagine residue corresponding to $Asn_{53}$. Removal of the C-terminal Tyr and Leu residues from $hirudin_{53-64}$ yielded a peptide, $hirudin_{53-62}$, with no reasonable inhibitory activity. Sulfated $hirudin_{53-64}$ - (Sulfo-$Tyr_{63}$$hirudin_{53-64}$) demonstrated a 10-fold increase in clotting time as compared to its unsulfated counterpart.

Based on these observations, we believe that the C-terminal amino acid residues of the intact hirudin peptide function, at least in part, in that protein's anti-thrombin activities. We have also found that a segment contained within those C-terminal residues in intact hirudin (in an opposite orientation to the hirudin peptide chain, i.e., from COOH to $NH_2$ terminus) is homologous to a segment contained within the proposed thrombin-binding domain of human thrombomodulin [D. Wen et al., "Human Thrombomodulin: Complete cDNA Sequence and Chromosome Localization of the Gene", Biochemistry, 26, pp. 4350-57 (1987)].

Thrombomodulin is an endothelial cell-surface glycoprotein which forms a stoichiometric complex with thrombin [C. T. Esman, "The Regulation of Natural Anticoagulant Pathways", Science, 235, pp. 1348-52 (1987)]. Identification of homology between the C-terminal segment in hirudin and residues 150-155 in the thrombomodulin precursor suggests that this segment in thrombomodulin participates in complex formation with thrombin. It has been observed previously that both hirudin-thrombin [J. L. Krstenansky and S. J. T. Mao, FEBS Lett., 211, pp. 10-16 (1987)] and thrombomodulin-thrombin complex formations yield a proteinase with a catalytic site. Together, these observations suggest that structural components in fibrinopeptides (in a productive mode) and in both thrombomodulin and hirudin (in an inhibitory mode) interact with a substrate-recognition site on thrombin which accommodates binding of fibrinogen. We believe that binding of hirudin peptides to thrombin leads to activation of protein C, thus further accelerating their in vivo anticoagulant properties via protein C degradation of Factors Va and Viiia. Because hirudin also impairs thrombin's catalytic activity it is unlikely to share in this property. Accordingly, we believe that the anticoagulant activity of the hirudin peptides of this invention may derive from binding of the peptide to a non-catalytic site of thrombin, thus yielding an increased $K_m$ for thrombin-catalyzed hydrolysis of fibrinogen.

## EXAMPLE 13

### Specificity Of Hirudin Peptides For Human Thrombin

The specificity of hirudin peptides for human α-thrombin was determined by comparing their relative inhibitory activities towards both human α-thrombin and bovine α-thrombin in a TT assay. Figure 7 demonstrates that Sulfo-$Tyr_{63}$-N-acetyl-$hirudin_{53-64}$ is 4 to 5 times more active as an inhibitor of human α-thrombin than bovine α-thrombin. Human α-thrombin and bovine α-thrombin are highly similar in structure, except for the presence of a unique lysine residue at position 149 of the β-chain in the human molecule. This residue defines the site of γ-autocatalytic degradation. We believe that Lys-149 of human α-thrombin is an important structural determinant for the binding of the hirudin peptides of this invention to thrombin.

EXAMPLE 14

Synergy Of Hirudin Peptides And Heparin In Anticoagulant Activity

The interaction of hirudin with thrombin has been shown to be blocked by the action of heparin/antithrombin III [S. R. Stone and J. Hofsteerige, "Effect of Heparin on the Interaction Between Thrombin and Hirudin", Eur. J. Biochem, 169, pp. 373-376, (1987)]. This result is attributed to the fact that both hirudin and heparin/antithrombin III form complexes with thrombin which block the catalytic site of that enzyme. We have observed that the hirudin peptides of this invention inhibit the fibrinogenolytic, but not amidolytic activity of thrombin. Accordingly, we carried out an assay to determine whether or not the hirudin peptides would act either additively or synergistically with heparin to anticoagulate human plasma. We observed synergy of hirudin peptides with heparin in APTT assays using pooled normal human plasma.

Figure 8 shows the results of a dose-dependent APTT assay using the hirudin peptide Sulfo-$Tyr_{63}hirudin_{53-64}$ alone, heparin alone, or the peptide and heparin combined. The APTT assay was carried out as in Example 12. Both heparin (Sigma, St. Louis, MO) and Sulfo-$Tyr_{63}hirudin_{53-64}$ (as prepared as in Example 10) were used at concentrations of 10 µg/ml. The results demonstrate that combinations of Sulfo-$Tyr_{63}hirudin_{53-64}$ and heparin yielded greater net increases in clotting time than those achieved by either molecule alone or from the predicted sum effect of their combined anticoagulant activities. Because heparin use is associated with hemorrhagic complications, such combinations are advantageous in achieving anticoagulant effects while using lower doses of heparin.

EXAMPLE 15

Measurement Of Thrombin Activity

We analyzed the hirudin peptides of this invention for inhibition of thrombin-catalyzed hydrolysis of the tripeptidyl p-nitroanilide substrate tosyl-Gly-Pro-Arg-p-nitroanilide (Chromozym TH, Boehringer-Mannheim, Indianapolis, In.) spectrophotometrically at 420 nm on a Cary 219 double-beam spectrophotometer. Reactions were prepared by mixing 10 µl of thrombin solution (bovine plasma thrombin, Calbiochem, Behring Diagnostics, La Jolla, CA, 0.1 mg/ml in 0.1 M Tris-HCl, pH 7.4, 0.15 M NaCl) with 1.0 ml of 0.1 M Tris-HCl, pH 7.4, 0.15 M NaCl buffer. We then added 25 µl of substrate solution (4 mg/ml tosyl-Gly-Pro-Arg-p-nitroanilide in the above buffer) and varying amounts of $hirudin_{45-64}$ (as prepared in Example 4) from a 1.0 mg/ml stock solution to a final fixed volume of 1.035 ml, with final concentrations of thrombin and substrate of $2.8 \times 10^{-8}$ M and $1.4 \times 10^{-4}$ M, respectively.

We found that at concentrations between $4.2 \times 10^{-6}$ M to $2.1 \times 10^{-7}$ M, $hirudin_{45-64}$ demonstrated no measurable inhibitory effect on thrombin-catalyzed hydrolysis of the artificial substrate. In contrast, 1.2 and $2.4 \times 10^{-8}$ M concentrations of intact hirudin led to a 48% and 73% inhibitory effect. Based upon these observations, we believe that peptides corresponding to the C-terminal 21 amino acids of hirudin, and smaller derivatives thereof, do not inhibit thrombin cleavage of small synthetic substrates. This is in complete agreement with our belief that the peptides of the present invention do not bind to the catalytic site of thrombin. Accordingly, we believe that the inhibitory effect on clotting time exhibited by the peptides of this invention may be attributed to their affinity for a site on thrombin other than the catalytic site.

EXAMPLE 16

Measurement Of In Vivo Anticoagulant Activity

We then measured the dose-dependent increase in clotting time of the hirudin peptides of this invention *in vivo* by intravenous injection of Sulfo-$Tyr_{63}$hirudin$_{53-64}$ into Balb/c mice (Charles River Laboratories, Boston, Mass.). Doses containing 10, 50, 100 and 250 µg of Sulfo-$Tyr_{63}$hirudin$_{53-64}$ were injected into the animals. After 5 minutes, the animals were bled. Blood was collected in a citrated vessel and plasma was isolated by centrifugation. The mouse plasma was assayed for activated partial thromboplastin times (APTT) by the methods described in Example 12. Figure 9 demonstrates that Sulfo-$Tyr_{63}$ hirudin$_{53-64}$ produced a dose-dependent increase *in vivo* in the APTT of mouse plasma.

## EXAMPLE 17

### Preparation Of A Polyethyleneglycol Conjugate Of Sulfo-$Tyr_{63}$Hirudin$_{53-64}$

We coupled a hirudin peptide of this invention to a pharmaceutically acceptable polymer in order to increase the half-life of the peptide. More specifically, we prepared a derivative of polyethyleneglycol, polyethyleneglycol N-succinimidyl succinate (SS-PEG; avg. MW = 5,000), by conventional methods [Abuchowski et al., Cancer Biochem. Biohys., 7, pp. 175-86 (1984)]. We then dissolved 100 µg of Sulfo-$Tyr_{63}$hirudin$_{53-64}$, as prepared in Example 10, in 200 µl of 20 mM sodium borate, pH 9.0 and reacted it with a 50-fold molar excess of SS-PEG. The reaction was let stand at room temperature overnight and then applied to reverse phase HPLC for purification and characterization.

Reverse-phase HPLC was performed using an Aquapore RP-300 $C_8$ column (0.46 x 3.0 cm) using an Applied Biosystems 150A chromatographic system. The column was equilibrated in water containing 0.1% TFA and developed with an increasing gradient of acteonitrile from 0 to 50% in a 0.085% TFA solvent over 45 minutes at a flow rate of 0.5 ml/min. The effluent stream was monitored at 214 nm for absorbance. We observed that the SS-PEG derivative of Sulfo-$Tyr_{63}$hirudin$_{53-64}$ elutes earlier than the underivatized form and is contained within a broader peak. Comparison of the anticoagulant activity of both the derivatized and underivatized forms of Sulfo-$Tyr_{63}$hirudin$_{53-64}$ revealed identical dose-dependencies for increase in APTT. Thus, the SS-PEG-Sulfo-$Tyr_{63}$hirudin$_{53-64}$ is an active derivative of the peptides of present invention that advantageously exhibits an expected increase in circulating half-life when compared to its underivatized counterpart.

## EXAMPLE 18

### Assay Of Inhibition Of Factors IXa And Xa

We indirectly measured the inhibition of Factor IXa by Sulfo-$Tyr_{63}$hirudin$_{53-64}$ based on our observations that Factor IXa catalyzes the activation of Factor X to Factor Xa; and that Sulfo-$Tyr_{63}$hirudin$_{53-64}$ does not inhibit the cleavage of a small chromogenic substrate, S-2222, by Factor Xa.

We prepared a Factor IXa/Factor X/phospholipid mixture from a Coatest Factor VIII assay kit (Kabivitrum, Helena Laboratories, Beaumont, TX) according to manufacturer's directions, in the presence of varying concentrations of Sulfo-$Tyr_{63}$hirudin$_{53-64}$ (0 to 66.7 µg/ml), as prepared in Example 10. We activated the mixtures by adding aliquots of 25 mM $CaCl_2$ and normal human plasma as recommended by the manufacturer. After the samples were incubated for 10 minutes at 37° C, we added 100 µl of S-2222 solution (Kabivitrum, Helena Laboratories, Beaumont, TX) to each sample. The reactions were stopped after 5 minutes by acidification. The extent of S-2222 cleavage was followed spectrophotometrically at 405 nm.

Figure 10 demonstrates that Sulfo-$Tyr_{63}$hirudin$_{53-64}$ exhibits a dose-dependent inhibition of S-2222 cleavage in this assay. Because this peptide does not inhibit Factor Xa cleavage of S-2222, this result shows that increasing amounts of peptide cause decreasing production of Factor Xa. Therefore, Sulfo-$Tyr_{63}$hirudin$_{53-64}$ must inhibit Factor IXa activation of Factor X. Inhibition of Factor IXa was complete at 6 x $10^{-6}$ M concentration of the peptide.

We assayed the inhibition of Factor Xa activation of prothrombin by Sulfo-$Tyr_{63}$hirudin$_{53-64}$ using SDS-

PAGE. We generated Factor Xa from a Factor IXa/X mixture in the presence of calcium phospholipid and diluted plasma as in the previous assay. The Factor Xa thus prepared was incubated with prothrombin, purified from normal human serum by the method of A. R. Thompson, J. Clin. Invest., 59, pp. 900-05 (1977), (130 µg/ml) in 315 µl of 0.05 M Tris-HCl, pH 7.5, 0.1 M NaCl containing either Sulfo-$Tyr_{63}hirudin_{53-64}$ (0 to 95 µg/ml) or hirudin (0 to 25 U/ml). Following a 60 minute incubation at 37° C, we removed a 30 µl aliquot, mixed it with 4X non-reducing SDS-PAGE sample buffer and analyzed the sample in a 10% acrylamide gel by SDS-PAGE. We stained the gel with Coomassie Blue following electrophoresis. Inhibition of Factor Xa activation of prothrombin was evaluated visually by inspection of the reaction products on the gel. Figure 11 illustrates the dose-dependent inhibition of prothrombin conversion to thrombin by increasing concentrations of Sulfo-$Tyr_{63}hirudin_{53-64}$.

Alternatively, samples prepared as described above were analyzed for resulting thrombin activity using Chromozym TH (Boehringer-Mannheim, Indianapolis, IN) as a substrate. A 5 µl aliquot of each sample was added to a cuvette containing 1.0 ml of 0.01 M Tris-HCl, pH 7.5, 0.15 M NaCl and 4 µg/ml Chromozym TH. Thrombin activity was monitored continuously at 420 nm. Analysis of thrombin activity in this manner demonstrated a four-fold reduction in esterolytic activity generated via Factor Xa activation of prothrombin at a Sulfo-$Tyr_{63}hirudin_{53-64}$ concentration of 70 µg/ml.

Taken together, these data demonstrate that Sulfo-$Tyr_{63}hirudin_{53-64}$ inhibits Factor IXa activation of Factor X, as well as Factor Xa activation of prothrombin, by interaction with a non-catalytic site in both cases. However, the level of inhibition of Factors IXa and Xa is about 10-fold less than the level of inhibition of thrombin. These results suggest that the structure in hirudin which interacts with Factors IXa and Xa is localized to the C-terminal segment, i.e., inhibition follows via interaction with a non-catalytic site(s). They also imply that various forms of hirudin which lack the sulfated $Tyr_{63}$ residue may not demonstrate the non-anti-thrombin anticoagulant properties of the natural leech protein.

## EXAMPLE 19

### Anti-Metastatic Activity Of Hirudin Peptides

The anti-metastatic activity of hirudin peptides, preferably, Sulfo-$Tyr_{63}hirudin_{53-64}$, may be assayed using sarcoma T241 cells [L. A. Liotta et al., " Nature 284, pp. 67-68 (1980)] and syngeneic C57BL/6 Mice (Jackson Laboratory, Bar Harbor, ME). The mice are injected either intravenously or subcutaneously with 0 - 250 g/kg of Sulfo-$Tyr_{63}hirudin_{53-64}$, as prepared according to Example 10, followed by intravenous injection of $10^4$-$10^6$ T241 tumor cells. After 15 days, the animal is terminated and lung tumor colonies are quantitated. Anti-metastic activity of the hirudin peptide is measured as percent reduction in tumor colonies compared to a placebo-treated control.

## EXAMPLE 20

### Peptidomimetic Analogs Of Hirudin Peptides

The peptides of this invention, preferably Sulfo-$Tyr_{63}hirudin_{53-64}$ may be used to produce semi-peptidic or non-peptidic peptidomimetic analogs, synthetic molecules which exhibit antithrombin and anticoagulant activities. These peptidominetic analogs display the inhibitory activity toward thrombin hydrolysis of fibrinogen, Factor IXa activation of Factor X and Factor Xa activation of prothrombin which characterize the hirudin peptides of this invention.

The peptidomimetic analogs of this invention, hereinafter referred to as "hirulogs", are represented by the following chemical structures:

Hirulog-1:

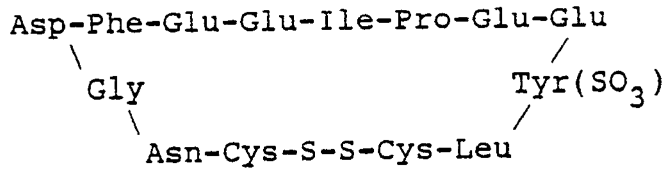

Hirulog-2:

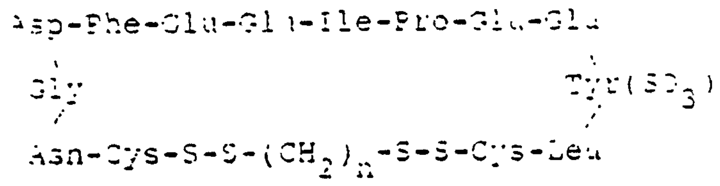

Hirulog-3:

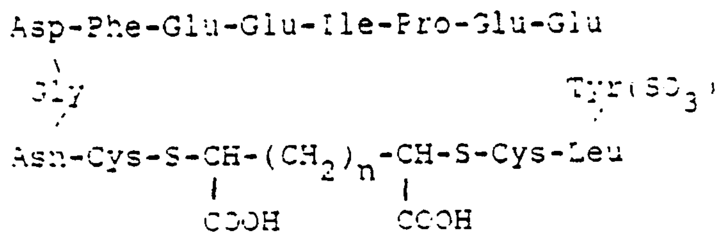

Hirulog-4:

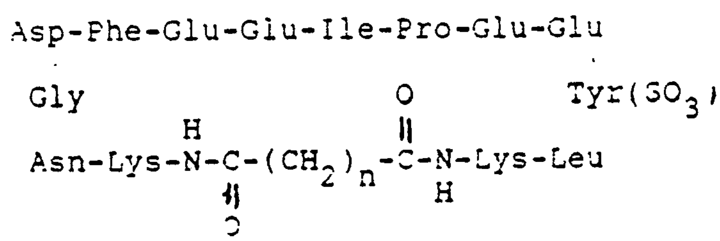

Hirulog-5:

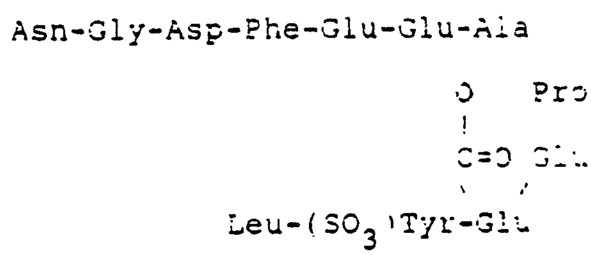

Hirulog-6:

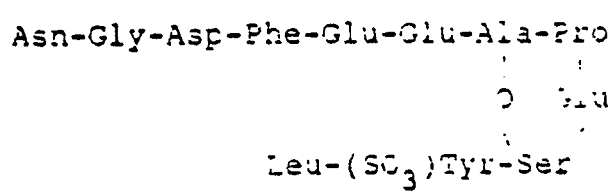

Hirulog-7:

```
Cys-Gly-Asp-Cys-Glu-Glu-Ile-Pro-Glu-Glu-Tyr(SO3)-Leu
|           |
S           S

 S-(CH2)2-S
```

Semi-peptidic peptidomimetic analogs of the hirudin peptides of this invention may be prepared to stabilize a loop, turn, or helical conformation of the parent peptide. For example, a loop structure is constructed by the addition of cysteinyl or lysyl residues at both the N- and C-terminal ends of Sulfo-$Tyr_{63}hirudin_{53-64}$. Terminal cysteinic residues are crosslinked by oxidation to produce Hirulog-1 (Figure 12a), oxidation with an aliphatic dithiol to produce Hirulog-2 (Figure 12b), or alkylation with aliphatic dihaloacetate or propionate to produce Hirulog-3 (Figure 12c). Terminal lysyl residues are crosslinked with any of a number of imidate agents which vary in spacer length or with dihydroxysuccinimidyl aliphatic reagents, resulting in production of Hirulog-4 (Figure 13).

A turn structure around Pro-8 of Sulfo-$Tyr_{63}hirudin_{53-64}$ is constrained by replacement of Ile-7 with chloroalanine, with or without concomitant replacement of Glu-9 or Glu-10 with (L) or (D)-serine. Peptidomimetic analogs containing chloroalanine alone would yield cross-linking of Glu-9 or Glu-10 to Ala-7 with a ketone linkage to produce Hirulog-5 (Figure 14a). Derivatives with serine at positions 9 or 10 would yield crosslinking via an ether linkage, producing Hirulog-6 (Figure 14b).

A helical structure in the peptidomimetic analogs of this invention can be constrained by substituting cysteinyl residues at position (n) and (n+3) of the hirudin peptide and crosslinking by either direct oxidation, oxidation with an aliphatic dithiol, or alkylation with an aliphatic dihalo acetate. For example, replacement of Asn-1 and Phe-4 of Sulfo-$Tyr_{63}hirudin_{53-64}$ with cvsteines and oxidation via ethanedithiol (Figure 15) would constrain a helical turn in the $NH_2$-terminal side of the derivative and, thus, seed a stable helical structure in the peptide derivative. This is exemplified by Hirulog-7.

Fully non-peptidic peptidomimetic analogs within this invention may also be produced, taking into consideration the above-described strategies relating to constrained peptide compounds.

## EXAMPLE 21

### Use Of Hirudin Peptides In Thrombus Imaging

Hirudin peptides, such as Sulfo-$Tyr_{63}hirudin_{53-64}$, may be modified by covalent attachment of $^{123}I$, $^{125}I$- or $^{111}In$-containing chemical groups. For example, the α-ammonium group of Sulfo-$Tyr_{63}$ $hirudin_{53-64}$ (as prepared in Example 10) may be reacted with $^{125}I$-Bolton-Hunter Reagent (New England Nuclear, Boston, MA.) in 0.1M sodium borate, pH 9. 0. The $^{125}I$-labelled peptide (with a specific radioactivity of >5 μCi/μg) is then desalted on a column of Biogel P2 which is equilibrated in a phosphate-buffered saline. The $^{125}I$-labelled peptide may then be tested in thrombin time and APTT assays to monitor any loss in antithrombin activity.

*Ex vivo* imaging of experimental thrombi is performed essentially as described by T. M. Palabrica et al., "Thrombus Imaging in a Primate Model with Antibodies Specific For an External Membrane Protein of Activated Platelets", manuscript submitted to *Proc. Natl. Acad. Sci. USA*, 86, pp. 1036-40 (1989). Specifically, imaging is performed in baboons with an external Ticoflex shunt between the femoral artery and femoral vein. An experimental thrombus is formed by placement of a segment of preclotted Dacron graft in the shunt. $^{125}I$-labelled Sulfo-$Tyr_{63}hirudin_{53-64}$ is injected in the venous part of the Ticoflex shunt. Serial anterior images are then obtained for 0.5 - 1 hr using an Ohio Nuclear Series 100 Gamma Camera with a PDP-11/34 computer. The kinetics of $^{125}I$-hirudin peptide uptake by the graft and blood pool are derived from the radionuclide images thus obtained.

The same technique may be used to obtain *ex vivo* images of a deep venous thrombus obtained by stasis in the femoral vein of baboons. Because Sulfo-$Tyr_{63}hirudin_{53-64}$ binds to thrombin with high specificity, the use of radiolabelled hirudin peptides allows precise *ex vivo* images of experimental thrombi.

Also, the small size of hirudin peptides, in contrast to native hirudin or antibodies to thrombin, provides the potential that the radiolabelled-peptide will yield images of platelet-bound thrombin and meizothrombin, as well as thrombin contained in the fibrin clot.

## EXAMPLE 22

### Comparison Of The Inhibitory Effect Of Low Doses Of Hirudin Peptides On Platelet Aggregation And Clotting Time

We prepared platelet-rich and platelet-poor plasma, according to the method of J. A. Jakubowski and N. G. Ardlie, "Modification of Human Platelet Function By A Diet Enriched in Saturated or Polyunsaturated Fat", Atherosclerosis, 31, pp. 335-44 (1978). Specifically, we collected blood from healthy human volunteers via a 21 gauge butterfly cannula into a 1/10 final volume of 3.8% trisodium citrate. All donors had avoided the use of medication of any kind for at least 2 weeks prior to blood collection Platelet-rich plasma was prepared by room temperature centrifugation of the citrated whole blood for 15 minutes at 100 x g in a Sorval rotor. We prepared platelet-poor plasma by centrifuging the citrated whole blood for 15 minutes at 12,000 x g.

We analyzed both the anticoagulant and antiplatelet activities of Sulfo-$Tyr_{63}$-N-acetyl-$hirudin_{53-64}$ over a concentration range of 0-0.55 µg/ml. Anticoagulant activity was assayed by measuring APTT as described in Example 12. Platelet aggregation was measured by adding Sulfo-$Tyr_{63}$-N-acetyl-$hirudin_{53-64}$ contained in 0.05 ml water to 0.4 ml of platelet-rich plasma prewarmed to 37° C. The mixture was stirred for 1 minute at 37° C and thrombin was then added to a final concentration of 0.4 U/ml. Aggregation was monitored turbidometrically over 4 minutes using a Biodata PAP4 4-channel Platelet Profiler (Biodata, Hatboro, PA).

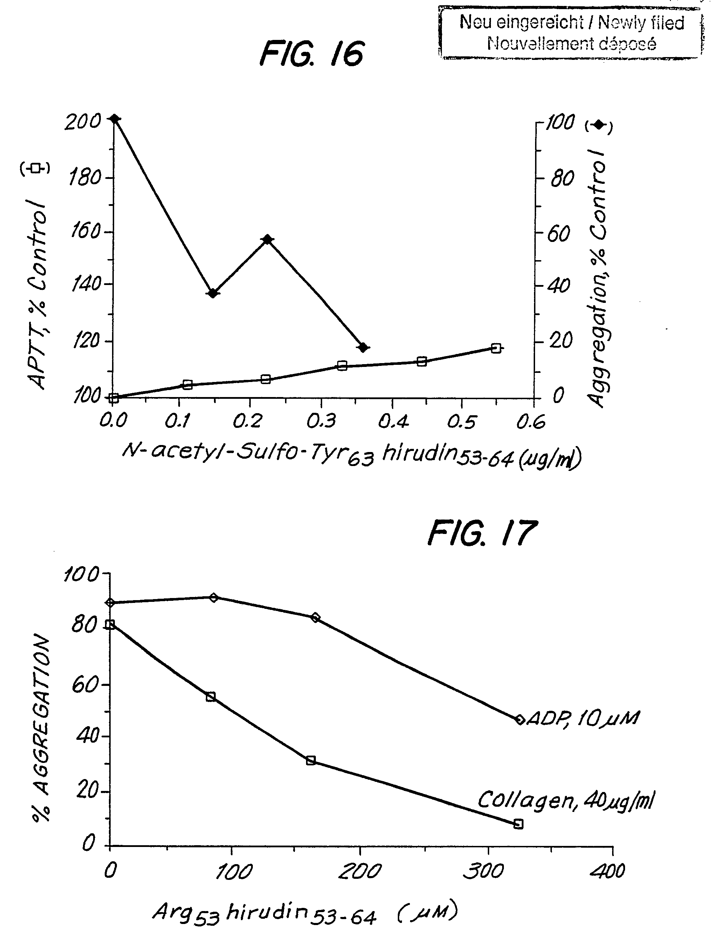

The anticoagulant activity of the highest concentration of Sulfo-$Tyr_{63}$-N-acetyl-$hirudin_{53-64}$ used in this assay yielded an APTT value that was less than 125% that of normal serum. In contrast, Sulfo-$Tyr_{63}$-N-acetyl-$hirudin_{53-64}$ inhibited platelet aggregation by 50% at concentrations of 0.15 - 0.25 µg/ml (Figure 16). This demonstrates that low doses of the hirudin peptides of this invention are effective in blocking platelet activation under conditions which do not therapeutically effect coagulation (i.e., an increase in APTT to 150-200% of control).

## EXAMPLE 23

### Comparison Of The Effects Of Hirudin, Sulfo-$Tyr_{63}Hirudin_{53-64}$ And $Hirudin_{53-64}$ On Endothelial Cells

We assayed the ability of $hirudin_{53-64}$ or Sulfo-$Tyr_{63}hirudin_{53-64}$ to inhibit thrombin-induced synthesis of platelet activating factor (PAF) using cultured human umbilical vein endothelial cells (HUVEC). HUVECs were extracted from human umbilical cords by collagenase digestion according to established procedures [M. A. Gimbrone,.Jr., "Culture of Vascular Endothelium", Prog. Hemost. Thromb., 3, pp. 1-28 (1976)]. We grew the HUVECs to confluence in a 96-well microtiter plate in the presence of [$^3$H]-acetate. Cells cultured in this manner produced [$^3$H]-acetyl-PAF, which can be quantitated by extraction of HUVEC membrane phospholipids.

We added either hirudin (0 - 1 µg/ml), $hirudin_{53-64}$ (0 - 200 µg/ml) or Sulfo-$Tyr_{63}hirudin_{53-64}$ (0 - 8 µg/ml) to the [$^3$H]-acetate loaded HUVECs 1 minute prior to the addition of thrombin (final concentration of 1 U/ml). We incubated the cells for 5 minutes and then removed the supernatant. We then added medium containing 0.1% gelatin, 50 mMacetic acid in methanol (2:1, v/v) to the HUVECs. We extracted and quantitated PAF using standard techniques [T. M. McIntyre et al., "Cultured Endothelial Cells Synthesize Both Platelet-Activating Factor and Prostacyclin in Response to Histamine, Bradykinin and Adenosine Triphosphate", J. Clin. Invest., 76, pp. 271-80.(1985)]. The $IG_{50}$ values for the three inhibitors is given below:

| Inhibitor | $IC_{50}$(nM) |
|---|---|
| hirudin | 14 |
| $hirudin_{53-64}$ | 11,364 |
| Sulfo-$Tyr_{63}hirudin_{53-64}$ | 281 |

We also examined the effect of Sulfo-$Tyr_{63}$-N-acetyl-$hirudin_{53-64}$ on thrombin-induced polymorphonuclear leukocyte (PMN) adhesion to HUVECs. We grew HUVECs in MEM containing 1% fetal calf serum to confluency in 24-well cluster plates. The medium was removed and the cells were washed two times with fresh, serum-free medium and incubated in the same medium for 10 - 30 minutes at 37° C to remove serum products. We added 1 ml of PMNs (2.5 x $10^6$/ml), which we pre-equilibrated at 37° C, to each well. We allowed the PMNs to settle onto the HUVEC monolayer for 2 minutes. We then added Sulfo-$Tyr_{63}$-N-acetyl-$hirudin_{53-64}$ (5 µg/ml) or saline to each well, immediately followed by the addition of thrombin (0.1 or 1.0 U/ml). The cells were incubated for 5 minutes at 37° C, washed twice and then examined by phase-contrast microscopy. Adherent PMNs were counted directly. The results indicated that PMN adherence was completely abolished in samples containing Sulfo-$Tyr_{63}$-N-acetyl-$hirudin_{53-64}$ and 0.1 U/ml thrombin and 75% inhibited in samples containing 1.0 U/ml thrombin.

## EXAMPLE 24

### The Effects Of N-Acetyl-$Arg_{53}$-$Hirudin_{53-64}$ On Collagen-Induced Platelet Aggregation

N-acetyl-$Arg_{53}hirudin_{53-64}$ was prepared by solid phase peptide synthesis and isolated to >95% purity by the procedures described in Example 1. This peptide contains an Arg-Gly-Asp sequence which is also prevalent in a wide number of adhesive proteins which interact with platelet glycoprotein IIb/IIIa [M. D. Pierschbacher and E. Ruoslahti, "Cell Attachment Activity of Fibronectin can be Duplicated by Small Synthetic Fragments of the Molecule", Nature, 309, pp. 30-33 (1984)].

We assessed the anticoagulant activity of N-acetyl-$Arg_{53}hirudin_{53-64}$ using an APTT assay as described in Example 12. N-acetyl-$Arg_{53}hirudin_{53-64}$ demonstrated 78.9% of the anticoagulant activity of N-acetyl-$hirudin_{53-64}$. We also examined the inhibition of platelet activation by N-acetyl-$Arg_{53}hirudin_{53-64}$ using the identical assay described in Example 22, except that platelet aggregation was stimulated by the addition of either collagen (40 µg/ml) or ADP (10 µM). Figure 17 demonstrates that N-acetyl-$Arg_{53}hirudin_{53-64}$ inhibited platelet aggregation in a dose-dependent manner, with an $IC_{50}$ of approximately 190 µg/ml for collagen-induced aggregation and 490 µg/ml for ADP-induced aggregation.

These results demonstrate that N-acetyl-$Arg_{53}hirudin_{53-64}$ is capable of blocking thrombin-mediated thrombosis, whether via cleavage of fibrinogen or activation of platelets, as well as platelet-dependent thrombosis caused by collagen, ADP, epinepherine, thromboxane $A_2$, or any other compound that activates platelets. In addition, the Arg-Gly-Asp sequence may serve to target this peptide to a thrombus and thus increase the local concentration of a thrombin inhibitor at that site.

## EXAMPLE 25

### Synthesis Of Hirudin Peptide Analogs Capable Of Forming Covalent Complexes With Thrombin

We synthesized the hirudin analog dinitrofluorobenzyl-Sulfo-$Tyr_{63}hirudin_{54-64}$ (DNFB-Sulfo-$Tyr_{63}hirudin_{54-64}$) by reacting stoichiometric quantities of Sulfo-$Tyr_{63}hirudin_{54-64}$ with dinitrodifluorobenzene (DNDFB) in dimethylformamide. We incubated the reaction mix for 24 hours at 22° C and then dried it under vacuum and redissolved in 0.1 % TFA in water. The resulting products were separated by HPLC chromatography employing an Aquapore RP-300 C8 octasilyl column (0.46 x 3.0 cm). The column was first equilibrated in 0.1% TFA in water (solvent A). After loading the sample, we developed the column with a 0 -

50% linear gradient of solvent B (0.085% TFA/70% acetonitrile) over 45 minutes. The effluent stream was monitored for adsorbance at 214 nm. The DNFB-Sulfo-$Tyr_{63}hirudin_{54-64}$ eluted at 48% solvent B.

We synthesized the hirudin analog $nitroanisole_{63}hirudin_{53-63}$ in a two-step method. First, we synthesized $methoxytyrosyl_{63}hirudin_{53-63}$ by the solid phase synthesis techniques, as described in Example 1, substituting Boc-O-methoxytyrosine resin for Boc-O-Leu resin in the synthesis. The peptide was cleaved from the resin and purified by HPLC on a Vydac C4 column, as described in Figure 1. We next nitrated the purified $methoxytyrosyl_{63}hirudin_{53-63}$ by adding to it an excess of tetranitromethane in 20 mM Tris-HCl, pH 8.0. The reaction was incubated at 27° C for 4 hours. The resulting products are lyophilized, redissolved in 20 mM ammonium bicarbonate and desalted on a Biogel P-4 column (1 x 30 cm) which was equilibrated and eluted in 20 mM ammonium bicarbonate. The separation of $nitroanisole_{63}hirudin_{53-63}$ was achieved by HPLC on an Aquapore RP-300 C8 octasilyl column (0.46 x 3.0 cm) as described above.

## EXAMPLE 26

### Demonstration Of Covalent Complex Formation Between Thrombin And DNFB-Sulfo-$Tyr_{63}hirudin_{54-64}$

We prepared DNFB-[$^{35}S$ ]-Sulfo-$Tyr_{63}hirudin_{54-64}$ following the procedure described in Example 25. We used Sulfo-$Tyr_{63}$ $hirudin_{54-64}$, which had been sulfated using $H_2[^{35}S]O_4$ during the sulfation procedure (see Example 10), as starting material.

We added a 10-fold molar excess of DNFB-[$^{35}S$]-Sulfo-$Tyr_{63}$ $hirudin_{54-64}$ to a solution containing 0.35 nmoles of human thrombin in phosphate buffered saline. After incubating the mixture for 3 hours, we performed SDS-polyacrylamide gel electrophoresis on the sample (12.5% acrylamide). Following autoradiography, a labeled band which corresponded to the size of thrombin was visualized. When we performed the binding step in the presence of a 50-or 500-fold excess of unlabeled N-acetyl-Sulfo-$Tyr_{63}hirudin_{54-64}$, formation of a covalent complex was blocked.

While we have hereinbefore presented a number of embodiments of this invention, it is apparent that our basic construction can be altered to provide other embodiments which utilize the processes and products of this invention. Therefore, it will be appreciated that the scope of this invention is to be defined by the claims appended hereto rather than the specific embodiments which have been presented hereinbefore by way of example.

## Claims

1. A peptide displaying anticoagulant activity, wherein said peptide is selected from the group consisting of:
(a) peptides characterized by a sequence of amino acids consisting substantially of the formula:
Asn-Gly-Asp-Phe-Glu-Glu-Ile-Pro-Glu-Glu-Tyr-X;
and
(b) D-retro forms of the peptides of (a);
wherein X is selected from the group consisting of COOH, Leu and Leu-Gln.

2. A peptide displaying anticoagulant activity, wherein said peptide is selected from the group consisting of:
(a) a peptide characterized by a sequence of amino acids consisting substantially of the formula:
Y-Phe-Glu-Glu-Ile-Pro-Glu-Glu-Tyr-Z;
and
b) D-retro forms of the peptides of (a);
wherein Y is selected from the group consisting of $NH_2$, an amino protecting group, at least the C-terminal portion of the amino acid sequence:
Val-Thr-Gly-Glu-Gly-Thr-Pro-Lys-Pro-Gln-Ser-His-Asn-Asp-Gly-Asp,
and at least the C-terminal portion of the amino acid sequence:
Val-Thr-Gly-Glu-Gly-Thr-Pro-Asn-Pro-Glu-Ser-His-Asn-Asn-Gly-Asp;
Z is selected from the group consisting of COOH, Leu and Leu-Gln; and the tyrosine residue contains a negatively charged side group.

3. The peptide according to claim 2, wherein the negatively charged side group is selected from the group consisting of sulfate, phosphate, carboxylate, sulfonate, phosphonate, carbonate, methylsulfonate and methylphosphonate.

4. The peptide according to claim 2, wherein the negatively charged side group is selected from the group consisting of sulfate and sulfonate.

5. The peptide according to claim 4, wherein Y is Asn-Gly-Asp, Z is Leu and said negatively charged side group is sulfate, said peptide being Sulfo-$Tyr_{63}$hirudin$_{53-64}$.

6. The peptide according to claim 4, wherein Y is Asn-Gly-Asp, Z is Leu and said negatively charged side group is sulfonate, said peptide being Sulfonyl-$Tyr_{63}$hirudin$_{53-64}$.

7. The peptide according to claim 1 or 2, wherein the amino terminal amino acid is N-acetylated.

8. The peptide according to claim 7, wherein said peptide is selected from the group consisting of Sulfo-$Tyr_{63}$-N-acetyl-hirudin$_{53-64}$ and N-acetyl-Sulfonyl-$Tyr_{63}$hirudin$_{53-64}$.

9. The peptide according to claim 1 or 2, wherein the amino terminal amino acid is dinitrofluorobenzylated.

10. A peptide displaying anticoagulant activity, said peptide being capable of forming a covalent bond with thrombin, wherein said peptide is selected from the group consisting of:
(a) a peptide characterized by a sequence of amino acids consisting essentially of the formula
: Y-Phe-Glu-Glu-Ile-Pro-Glu-Glu-W; and
(b) D-retro forms of the peptides of (a);
wherein Y is selected from the group consisting of $NH_2$, an amino protecting group, at least the C-terminal portion of the amino acid sequence:
Val-Thr-Gly-Glu-Gly-Thr-Pro-Lys-Pro-Gln-Ser-His-Asn-Asp-Gly-Asp, and at least the C-terminal portion of the amino acid sequence:
Val-Thr-Gly-Glu-Gly-Thr-Pro-Asn-Pro-Gln-Ser-His-Asn-Asn-Gly-Asp: and W is nitroanisole.

11. A peptide displaying anticoagulant and antiplatelet activity, wherein said peptide is selected from the group consisting of:
(a) a peptide characterized by a sequence of amino acids consisting essentially of the formula:
V-Arg-Gly-Asp-Phe-Glu-Glu-Ile-Pro-Glu-Glu-Tyr-Z; and
(b) D-retro forms of the peptides of (a);
wherein V is selected from the group consisting of $NH_2$, an amino protecting group, at least the C-terminal portion of the amino acid sequence:
Val-Thr-Gly-Glu-Gly-Thr-Pro-Lys-Pro-Gln-Ser-His-Asn, and and at least the C-terminal portion of the amino acid sequence:
Val-Thr-Gly-Glu-Gly-Thr-Pro-Asn-Pro-Gln-Ser-His-Asn; Z is selected from the group consisiting of COOH, Leu and Leu-Gln; and the tyrosine residue contains a negatively charged side group.

12. The peptide according to any one of claims 1, 2, 10 or 11, wherein said peptide is conjugated with a pharmacologically acceptable polymer.

13. The peptide according to claim 12, wherein said polymer is polyethyleneglycol N-succinimidyl succinate.

14. The peptide according to any one of claims 1, 2, 10 or 11, wherein said peptide is conjugated with a fibrinolytic agent.

15. A peptidomimetic analog of the peptide of claim 1 or 2, wherein said analog inhibits thrombin hydrolysis of fibrinogen and displays anticoagulant activity.

16. The peptidomimetic analog according to claim 15, wherein said analog is selected from the group consisting of hirulog-1, hirulog-2, hirulog-3, hirulog-4, hirulog-5, hirulog-6 and hirulog-7.

17. The peptidomimetic analog according to claim 15 or 16, wherein said peptidomimetic analog is conjugated with a fibrinolytic agent.

18. A pharmaceutically acceptable composition for increasing blood clotting time in a patient or in extracorporeal blood which comprises a pharmaceutically effective amount of at least one component selected from the group consisting of a peptide according to any one of claims 1 to 14, and a peptidomimetic analog according to any one of claims 15 to 17.

19. The composition according to claim 18, further comprising a compound selected from the group consisting heparin and low molecular weight heparin, wherein the dosage of said heparin or low molecular weight heparin is less than that required to achieve a desired result when said heparin or low molecular weight heparin is administered as a monotherapy.

20. The composition according to claim 19, wherein the dose of said heparin or low molecular weight heparin is less than about 2,500 to 5,000 units.

21. The composition according to any one of claims 18 to 20, wherein said composition further comprises a pharmaceutically effective amount of a fibrinolytic agent.

22. A method for increasing blood clotting time in a patient or in extracorporeal blood comprising the step of treating said patient or said extracorporeal blood in a pharmaceutically acceptable manner with a composition according to any one of claims 18 to 21.

23. A method for preventing or treating vascular disease in a patient, comprising the step of treating said patient in a pharmaceutically acceptable manner with a composition according any one of claims 18 to 21.

24. The use of a composition according to any one of claims 18 to 21 for the prevention or treatment of vascular disease.

25. A composition for determining the concentration of Factor IXa, Factor Xa, thrombin or mixtures thereof, in a biological sample, said composition comprising at least one peptide according to any one of claims 1 to 6.

26. A method for determining the concentration of Factor IXa, Factor Xa, thrombin or mixtures thereof, in a biological sample, said method comprising the step of contacting said sample with a composition according to claim 25.

27. A diagnostic kit for assaying the concentration of Factor IXa, Factor Xa, thrombin or mixtures thereof, in a biological sample, said kit comprising at least one peptide according to any one of claims 1 to 6.

28. A pharmaceutically acceptable composition for inhibiting the growth of metastatic tumors, wherein said composition comprises a pharmaceutically effective amount of at least one component selected from the group consisting of a peptide according to any one of claims 1 to 14 and a peptidomimetic analog according to any one of claims 15 to 17.

29. A method for inhibiting the growth of metastatic tumors in a patient, wherein said method comprises the step of treating said patient in a pharmaceutically acceptable manner with a composition according to claim 28.

30. The method according to claim 29, wherein said metastatic tumor is selected from the group consisting of carcinoma of the brain, carcinoma of the lung, carcinoma of the liver, osteocarcinoma and neoplastic cell carcinoma.

31. The peptide according to any one of claims 1, 2, 7, 9, 10 or 11, wherein said peptide is labeled with a radioisotope selected from the group consisting of $^{123}I$, $^{125}I$ and $^{111}In$.

32. A composition for ex vivo imaging of a fibrin or platelet thrombus in a patient comprising a peptide according to claim 31.

33. A method for ex vivo imaging of a fibrin or platelet thrombus in a patient comprising the steps of:

(a) administering to said patient a composition according to claim 32; and

(b) using detecting means to observe said composition.

34. A composition for coating the surface of an invasive device to be inserted into a patient, wherein said composition comprises at least one component selected from the group consisting of a peptide according to any one of claims 1 to 14 and a peptidomimetic analog according to any one of claims 15 to 17.

35. A method for coating the surface of an invasive device to be inserted into a patient, said method comprising the step of contacting said surface with a composition according to claim 34.

36. A pharmaceutical composition for increasing blood clotting time and inhibiting platelet activation in a patient, said composition comprising a pharmaceutically effective amount of a peptide according to claim 11.

37. A method for increasing blood clotting time and inhibiting platelet activation in a patient, wherein said platelet activation is induced by any agonist, said method comprising the step of treating said patient with a composition according to claim 36.

38. A pharmaceutical composition for inhibiting thrombin-induced platelet activation and thrombin-induced endothelial cell activation in a patient, said composition comprising a pharmaceutically effective low dosage amount of a peptide according to any one of claims 1 to 8, said low dosage being between about 0.0001 mg/kg body weight and about 0.099 mg/kg body weight.

39. A method for inhibiting thrombin-induced platelet activation and thrombin-induced endothelial cell activation in a patient, said method comprising the step of treating said patient with a composition according to claim 38.

40. A pharmaceutically acceptable composition for treating thrombin-induced inflammation in a patient, said composition comprising a pharmaceutically effective low dosage amount of a peptide according to any one of claims 1 to 8, said low dosage being between about 0.0001 mg/kg body weight and about 0.099 mg/kg body weight.

41. A method for treating thrombin-induced inflammation in a patient, said method comprising the step of administering to said patient a composition according to claim 40.

42. The method according to claim 41, wherein said thrombin-induced inflammation is caused by a disease selected from the group consisting of adult respiratory distress syndrome, septic shock, septicemia and reperfusion damage.

43. A method for sulfating a tyrosine residue of a peptide or a polypeptide, said method comprising the steps of:

(a) dissolving said peptide or polypeptide in an organic solvent;

(b) reacting said dissolved peptide or polypeptide with a dehydrating reagent to dehydrate the tyrosine residue of said peptide or polypeptide;

and

(c) sulfating said dehydrated tyrosine residue by adding sulfuric acid dropwise to said dissolved peptide or polypeptide until a precipitate forms.

44. The method according to claim 43, wherein said peptide is selected from the group consisting of N-acetyl-hirudin$_{53-64}$ and hirudin $_{53-64}$.

45. The method according to claim 43, wherein said organic solvent is dimethylformamide and said dehydrating reagent is dicyclohexylcarodiimide.

eingereicht / Newly filed
Nouvellement déposé

FIG. I

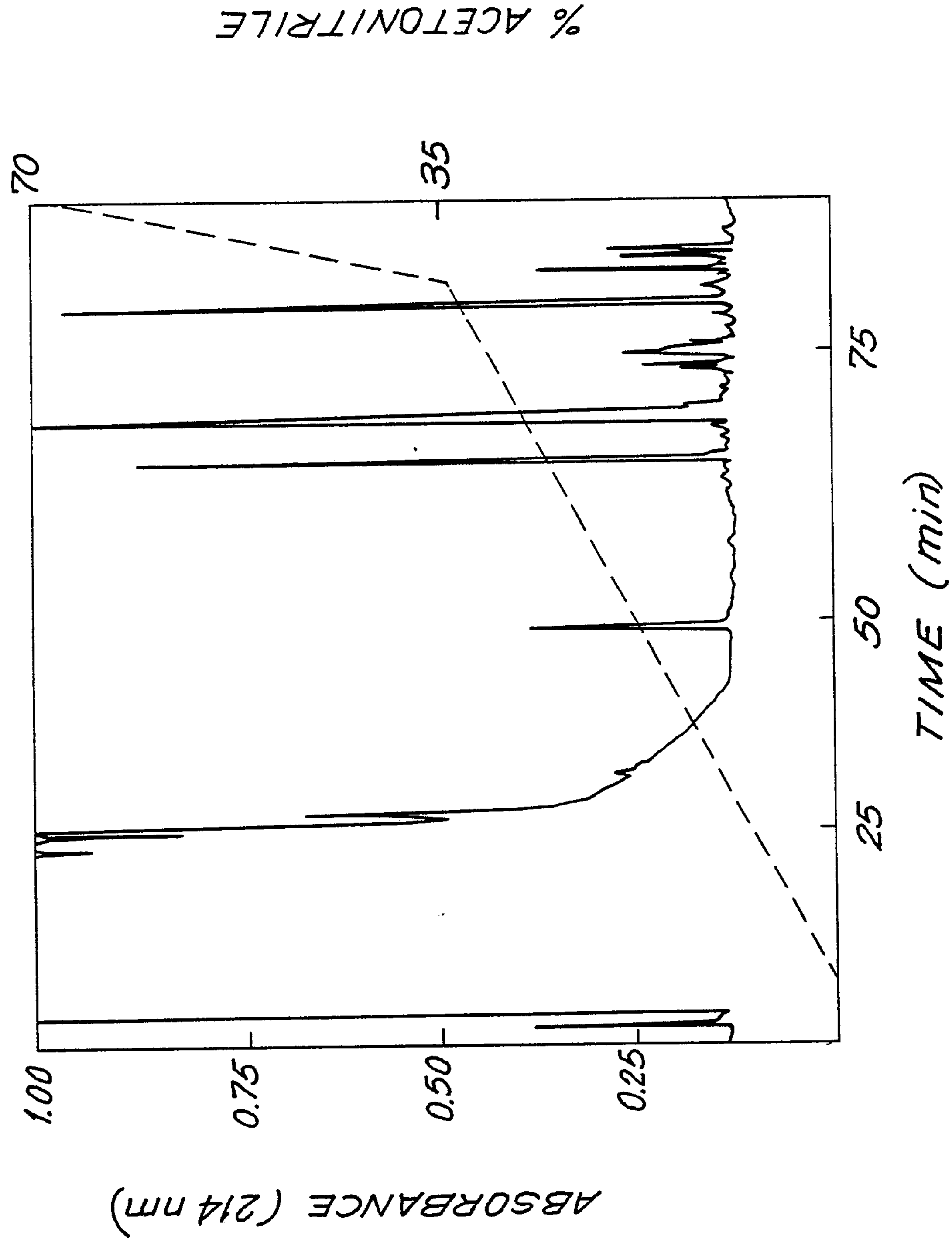

eingereicht / Newly filed
Nouvellement déposé

FIG. 2(a)

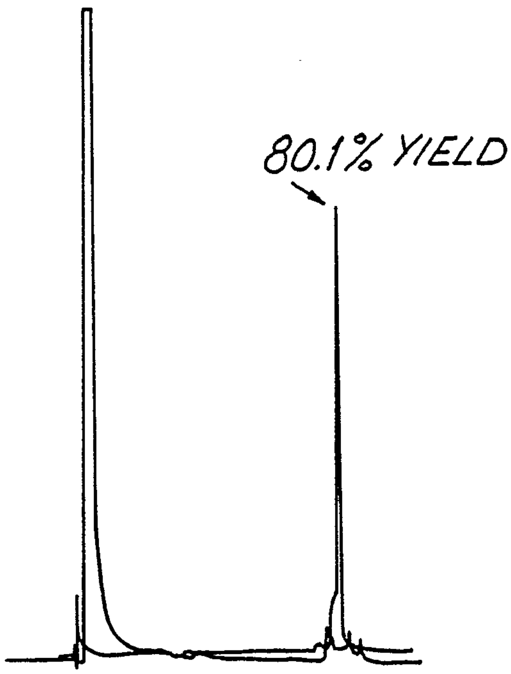


FIG. 2(b)

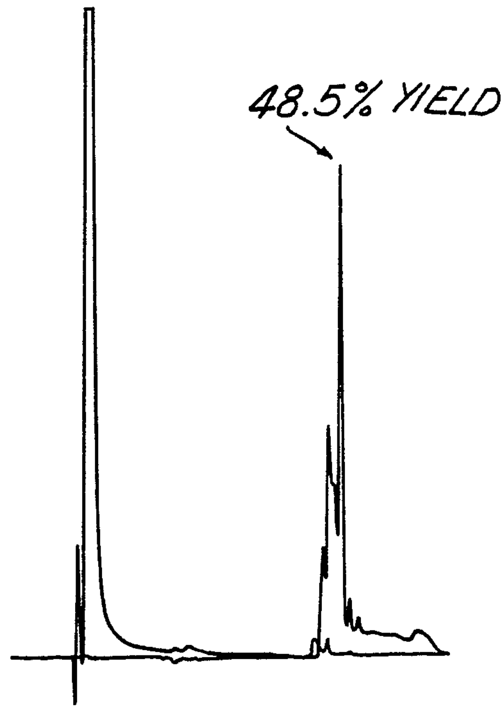


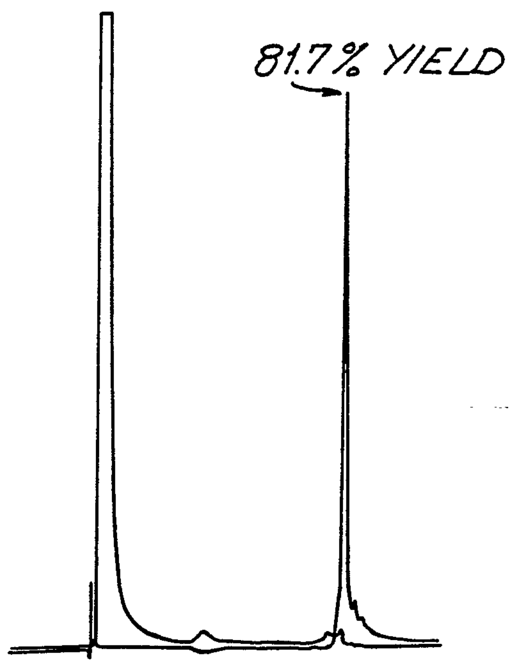


FIG. 2(c)

Neu eingereicht / Newly filed
Nouvellement déposé

FIG. 3

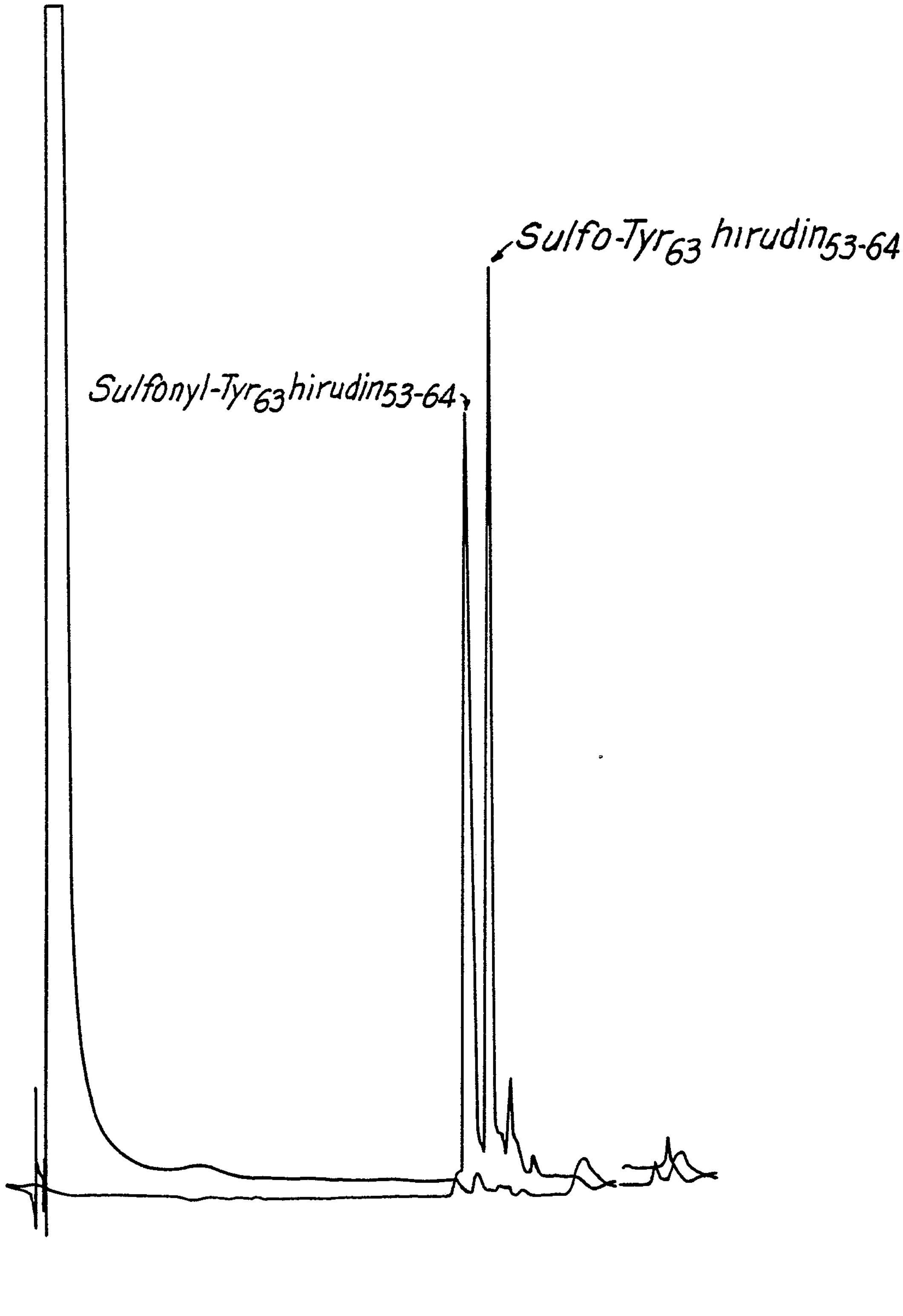

Eingereicht / Newly filed
Nouvellement déposé

FIG. 4

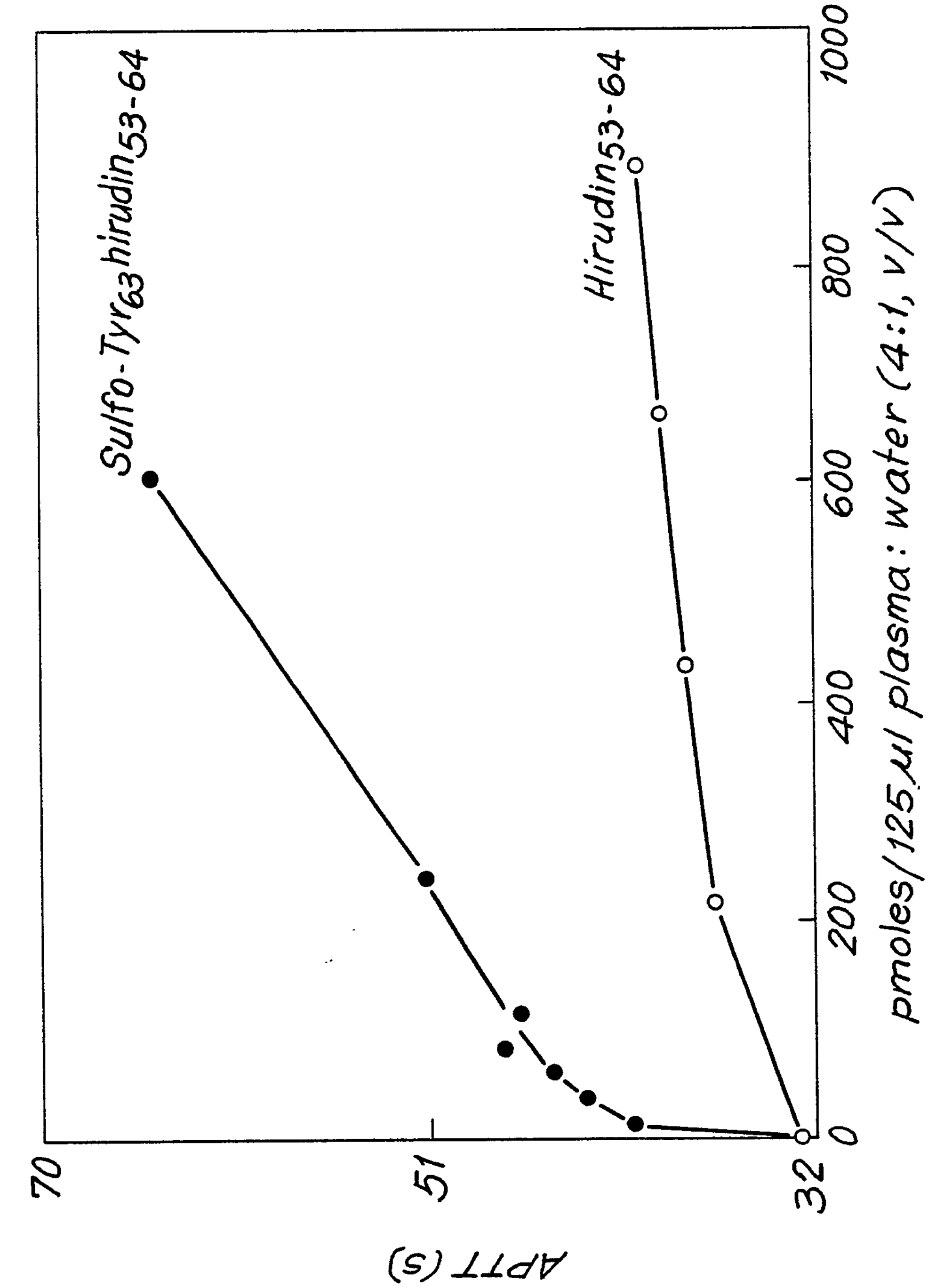

eingereicht / Newly filed
Nouvellement déposé

# FIG. 5

| Peptide | Position in Hirudin Sequence | Sequence | $MCT_{50}^{*}$ |
|---|---|---|---|
| $Hirudin_{53-64}$ | 53 - 64 | $NH_2$ - N G D F E E I P E E Y L - COOH | 0.77 |
| $Hirudin_{57-64}$ | 57 - 64 | $NH_2$ - E E I P E E Y L - COOH | >50 |
| des (Tyr-Leu) $Hirudin_{53-62}$ | 53 - 62 | $NH_2$ - N G D F E E I P E E - COOH | >50 |
| $Sulfo\text{-}Tyr_{63}$ $Hirudin_{53-64}$ | 53 - 64 | $NH_2$ - N G D F E E I P E E Y L - COOH<br>(Y) │ $SO_3$ | 0.08 |
| Control | 64 - 45 | $NH_2$ - L Y E E P I E E F D G N N H S E P N P T - COOH | >50 |

* $MCT_{50}$ is defined in nmoles peptide/125 µl of diluted plasma

Nouvellement déposé

FIG. 6

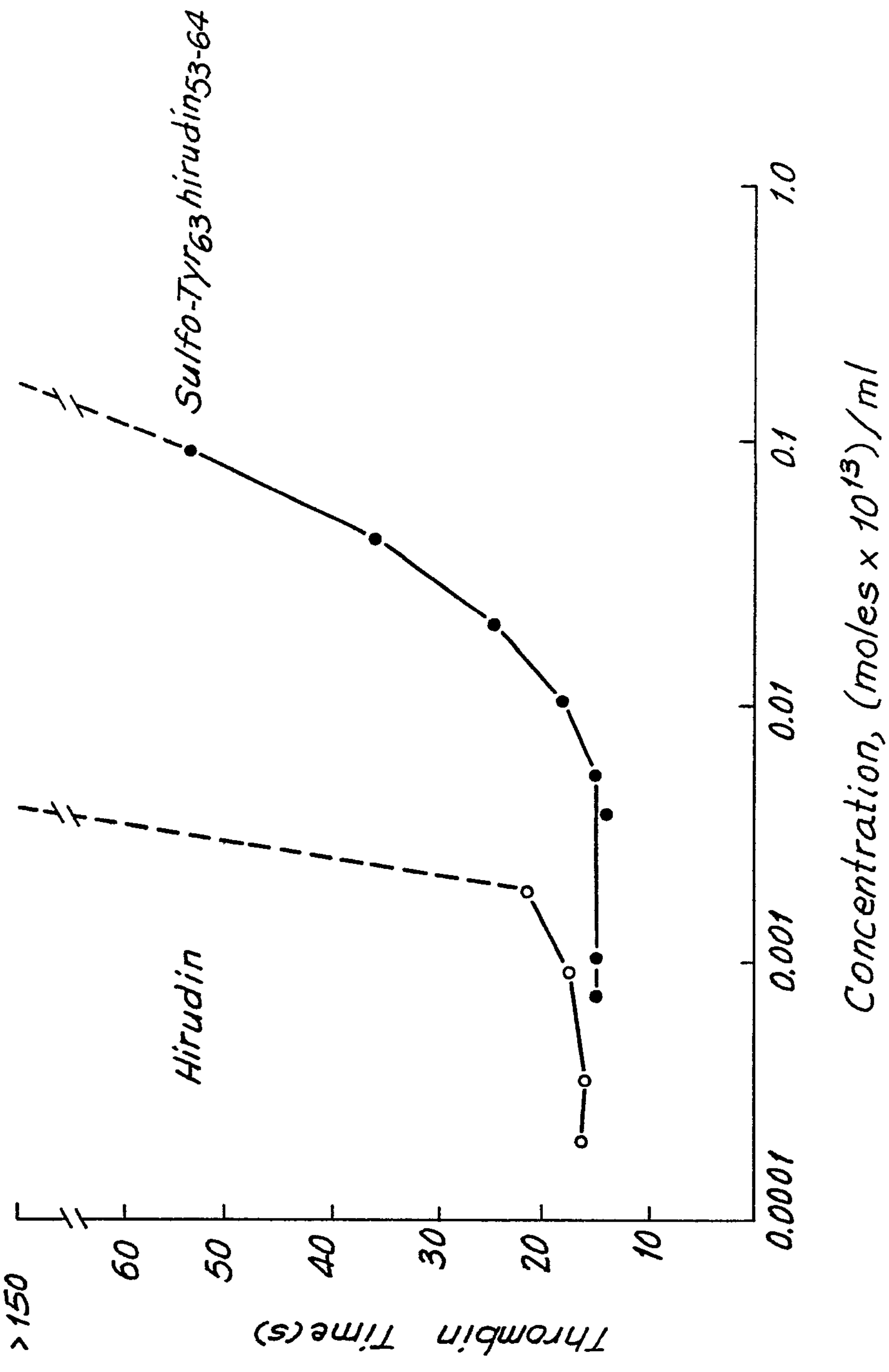

u eingereicht / Newly filed
Nouvellement déposé

FIG. 7

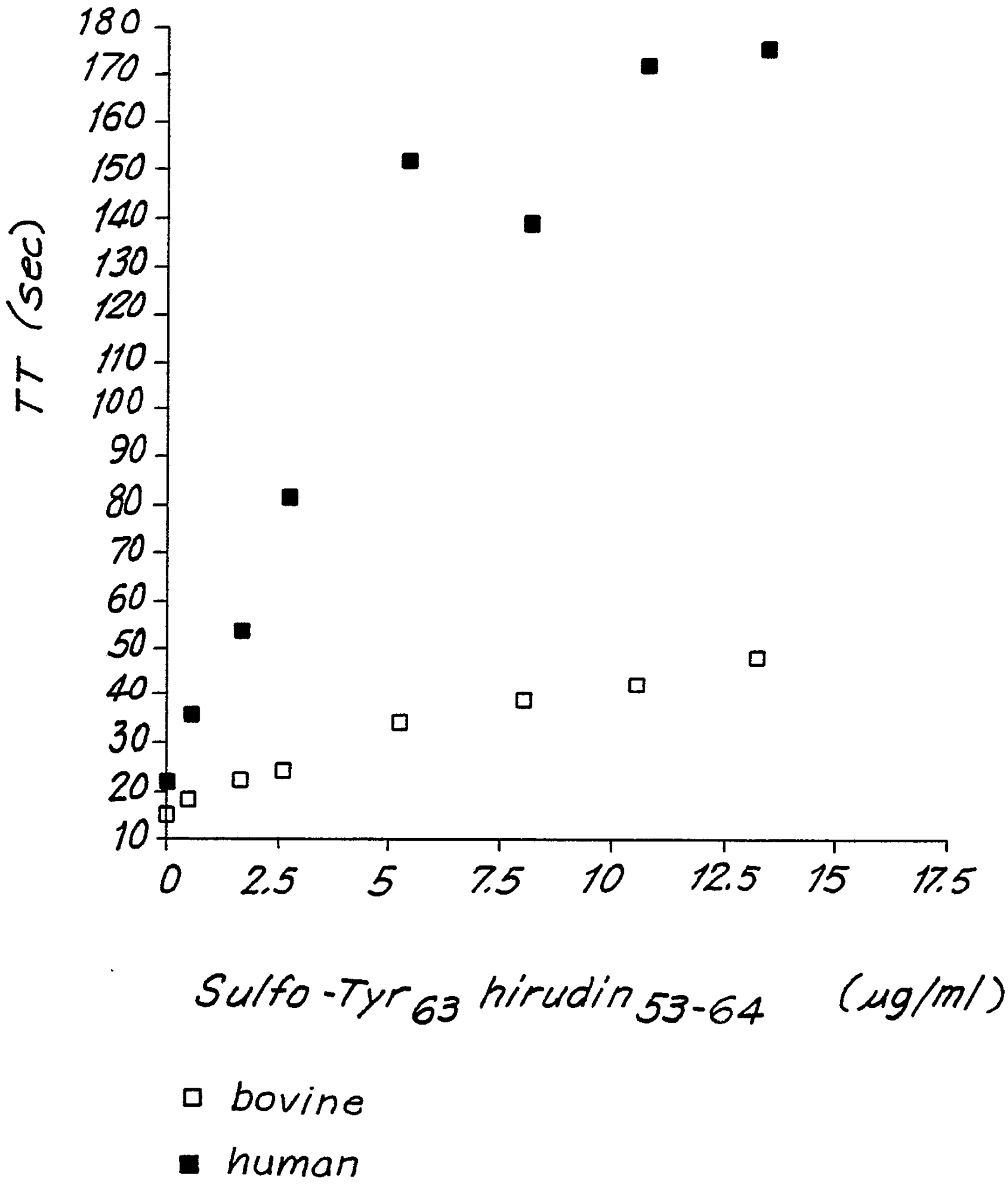

Neu eingereicht / Newly filed
Nouvellement déposé

FIG. 8

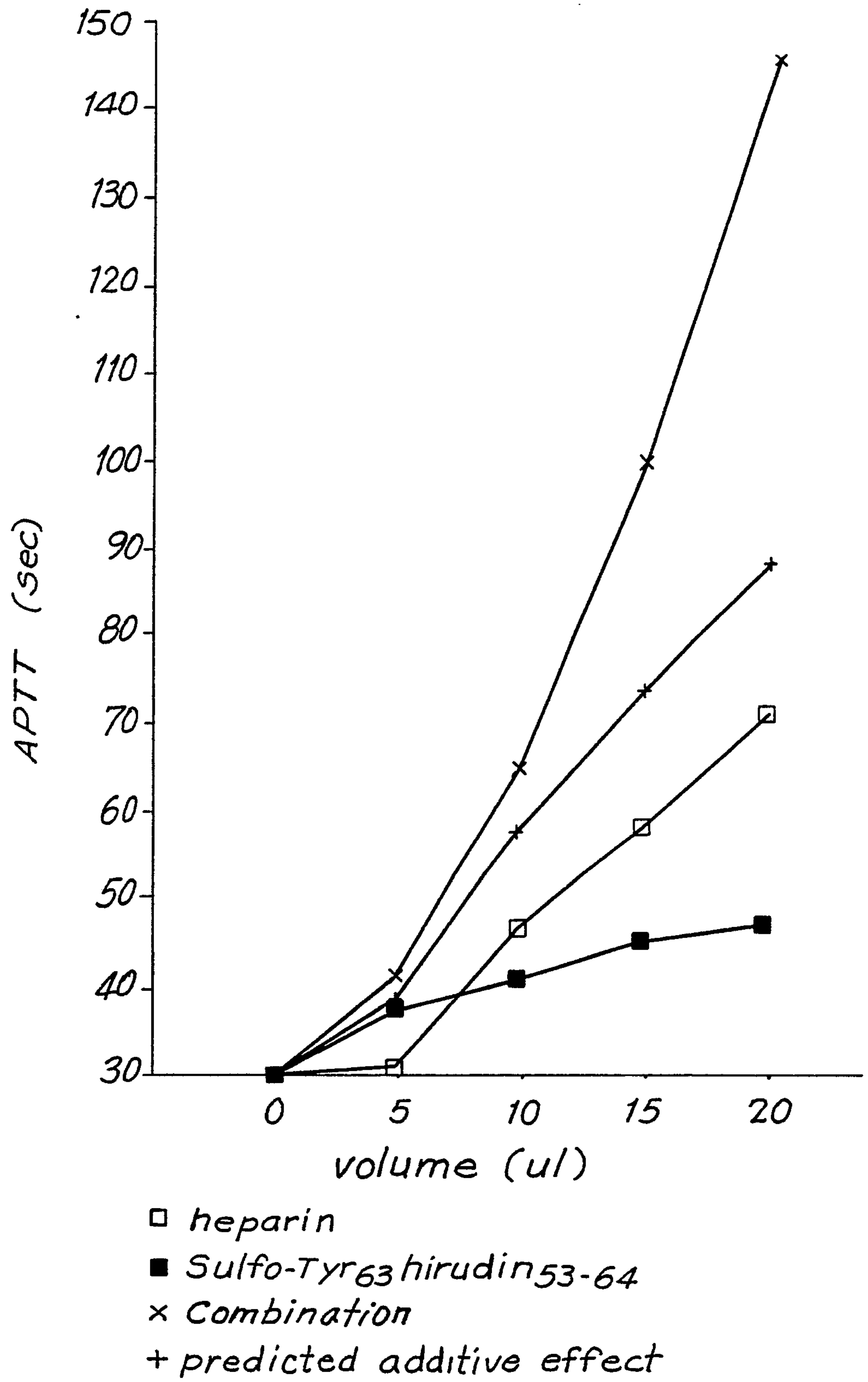

Eingereicht / Newly filed
Nouvellement déposé

FIG. 9

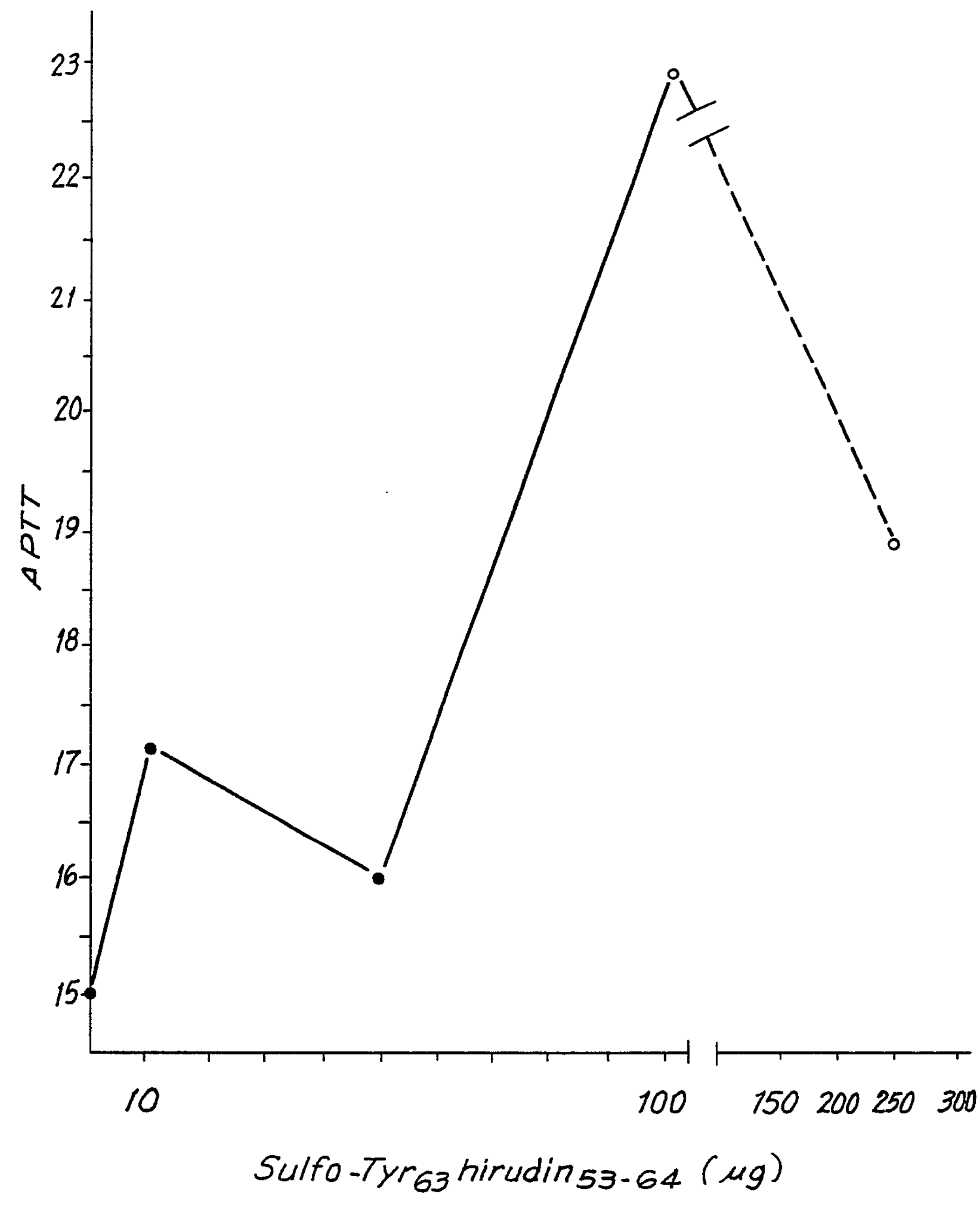

eu eingereicht / Newly filed
Nouvellement déposé

FIG. 10

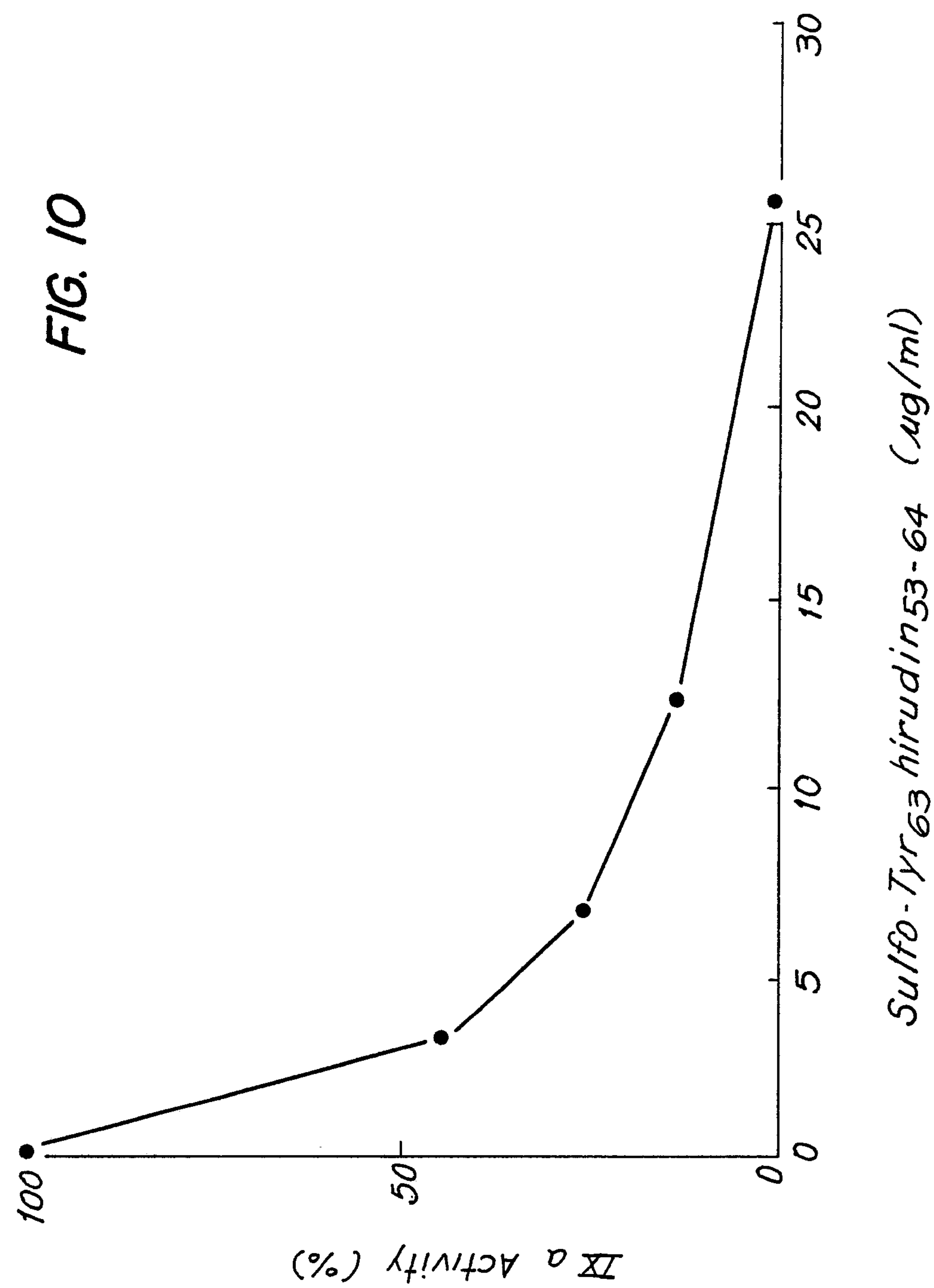

eingereicht / Newly filed
Nouvellement déposé

FIG. 11

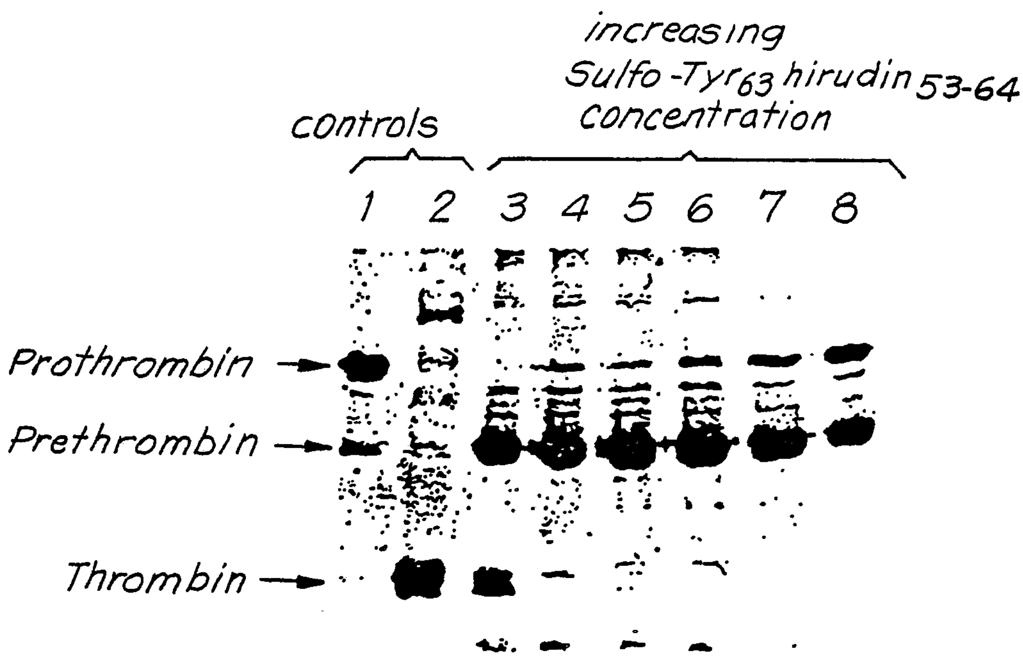

ngereicht / Newly filed,
uvellement déposé

# FIG. 12

```
A.
  Cys-Asn-Gly-Asp-Phe-Glu-Glu-Ile-Pro-Glu-Glu-Tyr(SO3)-Leu-Cys
   |                                                          |
   SH                                                         SH

                                  ↓ oxidation

                   Asp-Phe-Glu-Glu-Ile-Pro-Glu-Glu
                      \                          /
 Hirulog-1            Gly                   Tyr(SO3)
                        \                    /
                         Asn-Cys-S-S-Cys-Leu


B.
  Cys-Asn-Gly-Asp-Phe-Glu-Glu-Ile-Pro-Glu-Glu-Tyr(SO3)-Leu-Cys
   |                                                          |
   SH                                                         SH

                               ↓ oxidation/
                               ↓ aliphatic dithiol

                   Asp-Phe-Glu-Glu-Ile-Pro-Glu-Glu
                   |                             |
 Hirulog-2         Gly                           Tyr(SO3)
                   |                             |
                   Asn-Cys-S-S-(CH2)n-S-S-Cys-Leu


C.

  Cys-Asn-Gly-Asp-Phe-Glu-Glu-Ile-Pro-Glu-Glu-Tyr(SO3)-Leu-Cys
   |                                                          |
   SH                                                         SH

                                 ↓ alkylation

                   Asp-Phe-Glu-Glu-Ile-Pro-Glu-Glu
                   |                             |
 Hirulog-3         Gly                           Tyr(SO3)
                   |                             |
                 Asn-Cys-S-CH-(CH2)n-CH-S-Cys-Leu
                           |         |
                          COOH      COOH
```

Eingereicht / Newly filed
Nouvellement déposé

FIG. 13

A.

Lys-Asn-Gly-Asp-Phe-Glu-Glu-Ile-Pro-Glu-Glu-Tyr($SO_3$)-Leu-Lys

imidate

Hirulog-4

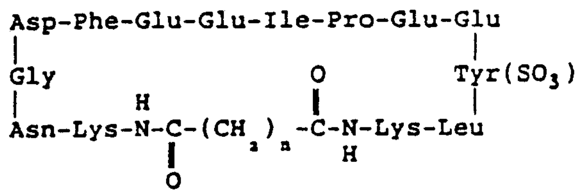

FIG. 14

A.

Asn-Gly-Asp-Phe-Glu-Glu-Ala(Cl)-Pro-Glu-Glu-Tyr($SO_3$)-Leu

Hirulog-5

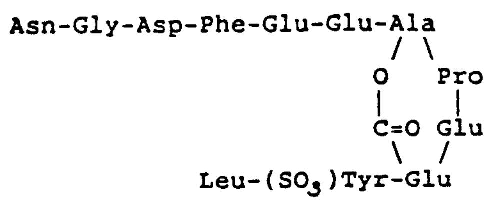

B.

Asn-Gly-Asp-Phe-Glu-Glu-Ala(Cl)-Pro-Glu-Ser-Tyr($SO_3$)-Leu

Hirulog-6

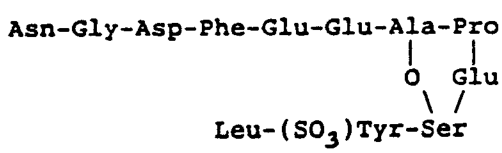

FIG. 15

Cys-Gly-Asp-Cys-Glu-Glu-Ile-Pro-Glu-Glu-Tyr($SO_3$)-Leu

HS-$(CH_2)_2$SH

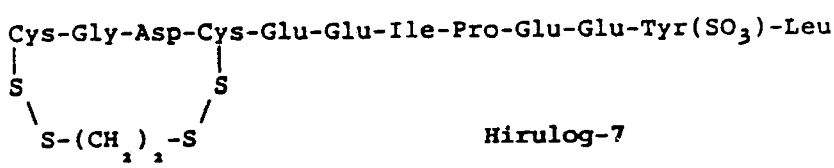

Hirulog-7

Neu eingereicht / Newly filed
Nouvellement déposé

FIG. 16

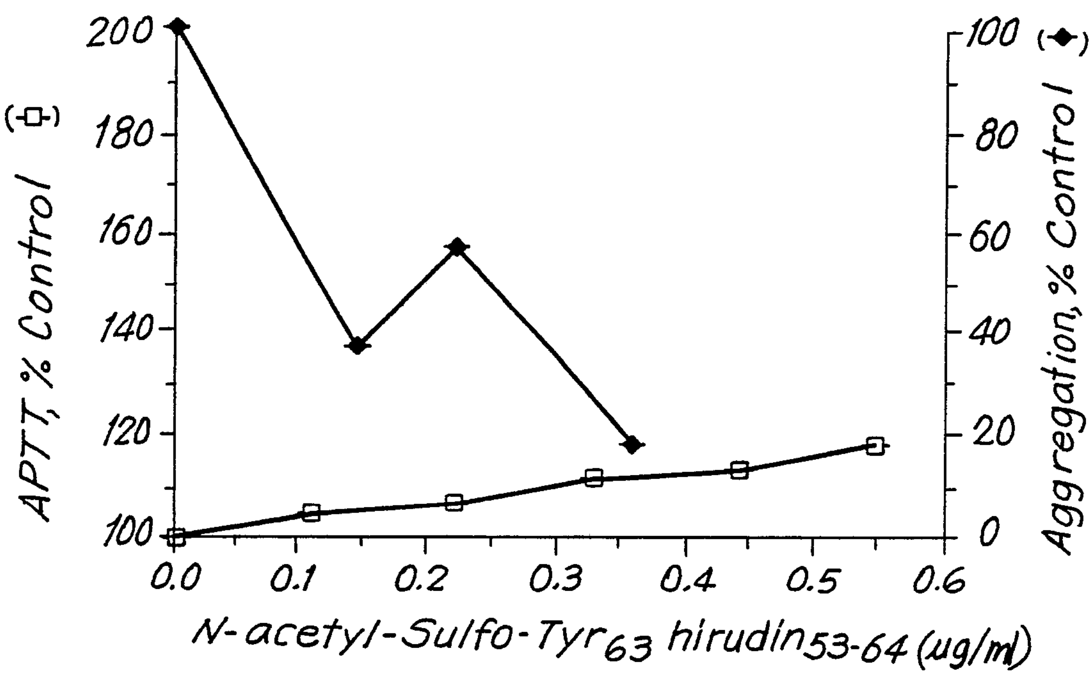


FIG. 17